# EUROPEAN PATENT SPECIFICATION

(11) **EP 4 291 296 B1**
(45) Date of publication and mention of the grant of the patent: **25.03.2026**
(21) Application number: 22752421.2
(22) Date of filing: 02.02.2022
(51) Int. Cl.: A61N 1/36, A61N 1/05, A61N 1/375, A61B 17/34, A61N 1/378, A61L 29/16, A61L 29/14

(54) **INTEGRATED TUBE SHEATH**
INTEGRIERTE SCHLAUCHHÜLLE
GAINE TUBULAIRE INTÉGRÉE

(30) Priority: 09.02.2021 US 202163147362 P
(43) Date of publication of application: 20.12.2023
(73) Proprietor: Cochlear Limited, Macquarie University, New South Wales 2109 (AU)
(72) Inventor: PAWSEY, Nicholas Charles Kendall, Macquarie University, New South Wales 2109 (AU); MANOUCHEHRI, Shahram, Macquarie University, New South Wales 2109 (AU)
(74) Representative: Reddie & Grose LLP
(86) International application number: PCT/IB2022/050916
(87) International publication number: WO 2022/172135

(56) References cited:
- EP-A2- 1 604 626
- WO-A1-2015/013120
- US-A1- 2003 069 613
- US-A1- 2008 082 141
- US-A1- 2008 082 141
- US-A1- 2008 154 339
- US-A1- 2017 333 699
- US-A1- 2019 224 482
- US-A1- 2019 282 803
- US-B2- 7 328 072

## Description

### BACKGROUND

### Field of the Invention

The present invention relates generally to tube sheaths for implantable stimulation assemblies for implantable medical devices.

### Related Art

Medical devices have provided a wide range of therapeutic benefits to recipients over recent decades. Medical devices can include internal or implantable components/devices, external or wearable components/devices, or combinations thereof (e.g., a device having an external component communicating with an implantable component). Medical devices, such as traditional hearing aids, partially or fully-implantable hearing prostheses (e.g., bone conduction devices, mechanical stimulators, cochlear implants, *etc.*), pacemakers, defibrillators, functional electrical stimulation devices, and other medical devices, have been successful in performing lifesaving and/or lifestyle enhancement functions and/or recipient monitoring for a number of years.

The types of medical devices and the ranges of functions performed thereby have increased over the years. For example, many medical devices, sometimes referred to as "implantable medical devices," now often include one or more instruments, apparatus, sensors, processors, controllers or other functional mechanical or electrical components that are permanently or temporarily implanted in a recipient. These functional devices are typically used to diagnose, prevent, monitor, treat, or manage a disease/injury or symptom thereof, or to investigate, replace or modify the anatomy or a physiological process. Many of these functional devices utilize power and/or data received from external devices that are part of, or operate in conjunction with, implantable components.

US 2008/082141 A1 relates to an electrode assembly comprising a low-profile, low-volume elongate electrode carrier and a corresponding guide tube for introducing the carrier into the cochlea to place electrodes disposed at the distal end of the carrier at desired locations along the tonotopically-mapped cochlea. The electrode assembly facilitates intra- and extra-cochlea atraumatic implantation of the unobtrusive electrode carrier of the present invention thereby minimizing adverse impact to natural auditory functioning. For example, the electrode assembly may be utilized to implant the low-profile, low-volume elongate electrode carrier into the scala tympani without damaging the delicate structures of the cochlea and without interfering with the natural hydrodynamic nature of the cochlea such as the natural flow of perilymph in the cochlea canals. In one particular embodiment, the carrier is pre-curved to attain a perimodiolar position in the scala tympani to facilitate accurate delivery of electrical stimulation with a minimum stimulation current and power consumption.

### SUMMARY

The invention is defined by the appended claims.

In one aspect, an implantable medical device system is provided. The implantable medical device system comprises: a stimulation arrangement comprising an elongate stimulation assembly disposed at a distal end of the stimulation arrangement, wherein the elongate stimulation assembly is configured to be inserted into a cochlea of a recipient; and an integrated tube sheath configured to be permanently disposed around and not removable from an outer circumference of the stimulation arrangement.

In another aspect, a method is provided. The method comprises: forming a surgical opening in a body of a recipient of an implantable medical device system; inserting a stimulation arrangement comprising an elongate stimulation assembly through the surgical opening, wherein a distal portion of the stimulation arrangement includes an elongate stimulation assembly, and wherein an integrated tube sheath is disposed around an outer circumference of stimulation assembly; positioning a distal end of the stimulation arrangement and a distal end of the integrated tube sheath into a cavity within the body of the recipient; sliding the elongate stimulation assembly relative to the integrated tube sheath to insert the elongate stimulation assembly into the cavity; and following insertion of the elongate stimulation assembly into the cavity, closing the surgical opening in the body with the elongate stimulation assembly and the integrated tube sheath within the body of the recipient.

In another aspect an implantable medical device system is provided. The implantable medical device system comprises: a stimulation arrangement comprising an elongate stimulation assembly configured to be inserted into a cavity of a recipient; an implant body connected to a proximal end of the stimulation arrangement; and an elongate tube sheath disposed around an outer circumference of the elongate stimulation assembly during insertion of the stimulation assembly into the cavity, wherein the elongate tube sheath is slideably engaged with an outer surface of the stimulation arrangement enabling longitudinal advancement of the stimulation assembly relative to the elongate tube sheath, and wherein, following insertion of the elongate stimulation assembly into the cavity, the elongate tube sheath is configured to be removed from the cavity and remain implanted in the recipient between the stimulation assembly and the implant body.

In another aspect an implantable medical device system is provided. The implantable medical device system comprises: a stimulation arrangement comprising an elongate stimulation assembly configured to be inserted into a cavity of a recipient; an implant body connected to a proximal end of the stimulation arrangement; and an elongate tube sheath disposed around an outer circumference of the elongate stimulation assembly during insertion of the stimulation assembly into the cavity, wherein the elongate tube sheath is slideably engaged with an outer surface of the stimulation arrangement enabling longitudinal advancement of the stimulation assembly relative to the elongate tube sheath, and wherein, following insertion of the elongate stimulation assembly into the cavity, at least a distal portion of the elongate tube sheath is configured to remain permanently disposed around a proximal region of the stimulation assembly within the cavity.

In another aspect, a method is provided. The method comprises: inserting an elongate stimulation assembly and into a cochlea of a recipient, wherein a elongate tube sheath is disposed around an outer surface of the stimulation assembly, and wherein the stimulation assembly is connected to an elongate transition region; and following insertion into the cochlea, sliding the elongate tube sheath from the stimulation assembly onto the transition region.

### BRIEF DESCRIPTION OF THE DRAWINGS

Embodiments of the present disclosure are described herein in conjunction with the accompanying drawings, in which:
FIG. 1A is a schematic diagram illustrating a cochlear implant system comprising an integrated tube sheath, in accordance with certain embodiments presented herein;
FIG. 1B is a side view of a recipient wearing a sound processing unit of the cochlear implant system of FIG. 1A;
FIG. 1C is a schematic view of components of the cochlear implant system of FIG. 1A;
FIGs. 1D is a block diagram of the cochlear implant system of FIG. 1A;
FIGs. 2A, 2B, 2C, and 2D are schematic diagram illustrating cross-sectional views of an integrated tube sheath and associated stimulation arrangement, in accordance with certain embodiments presented herein;
FIGs. 3A and 3B are schematic diagram illustrating cross-sectional views of another integrated tube sheath and associated stimulation arrangement, in accordance with certain embodiments presented herein;
FIG. 4 is a schematic diagram illustrating cross-sectional views of another integrated tube sheath and associated stimulation arrangement, in accordance with certain embodiments presented herein;
FIG. 5 is a schematic diagram illustrating cross-sectional views of another integrated tube sheath and associated stimulation arrangement, in accordance with certain embodiments presented herein;
FIGs. 6A and 6B are schematic diagram illustrating cross-sectional views of another integrated tube sheath and associated stimulation arrangement, in accordance with certain embodiments presented herein;
FIGs. 7A and 7B are schematic diagram illustrating cross-sectional views of another integrated tube sheath and associated stimulation arrangement, in accordance with certain embodiments presented herein;
FIGs. 8A and 8B are schematic diagram illustrating cross-sectional views of another integrated tube sheath and associated stimulation arrangement, in accordance with certain embodiments presented herein;
FIGs. 9A and 9B are schematic diagram illustrating cross-sectional views of another integrated tube sheath and associated stimulation arrangement, in accordance with certain embodiments presented herein;
FIGs. 10A and 10B are schematic diagram illustrating cross-sectional views of another integrated tube sheath and associated stimulation arrangement, in accordance with certain embodiments presented herein;
FIGs. 11A and 11B are schematic diagram illustrating cross-sectional views of another integrated tube sheath and associated stimulation arrangement, in accordance with certain embodiments presented herein;
FIG. 12 is a schematic diagram illustrating a vestibular implant system comprising an integrated tube sheath, in accordance with certain embodiments presented herein;
FIG. 13 is a flowchart of an example method, in accordance with certain embodiments presented herein; and
FIG. 14 is a flowchart of an example method, in accordance with certain embodiments presented herein.

### DETAILED DESCRIPTION

Presented herein are integrated tube sheaths (tube sheaths) for implantable stimulation assemblies, such as pre-curved perimodiolar stimulation assemblies (electrode arrays). An integrated tube sheath is configured to be disposed around, and is slideably engaged with, an outer surface of an associated stimulation assembly. The integrated tube sheath is configured to retain the stimulation assembly in a straight arrangement for implantation of the stimulation assembly into a recipient. Following implantation of the stimulation assembly, the integrated tube sheath is configured to remain implanted in the recipient.

Merely for ease of description, the integrated tube sheaths presented herein are primarily described with reference to a specific implantable medical device system, namely a cochlear implant system. However, it is to be appreciated that the techniques presented herein may also be implemented by other types of implantable medical devices. For example, the techniques presented herein may be implemented by other auditory prosthesis systems that include one or more other types of auditory prostheses, such as middle ear auditory prostheses, bone conduction devices, direct acoustic stimulators, electro-acoustic prostheses, auditory brain stimulators, combinations or variations thereof, *etc.* The techniques presented herein may also be used with tinnitus therapy devices, vestibular devices (e.g., vestibular implants), visual devices (i.e., bionic eyes), sensors, pacemakers, drug delivery systems, defibrillators, functional electrical stimulation devices, catheters, seizure devices (e.g., devices for monitoring and/or treating epileptic events), sleep apnea devices, electroporation devices, *etc.*

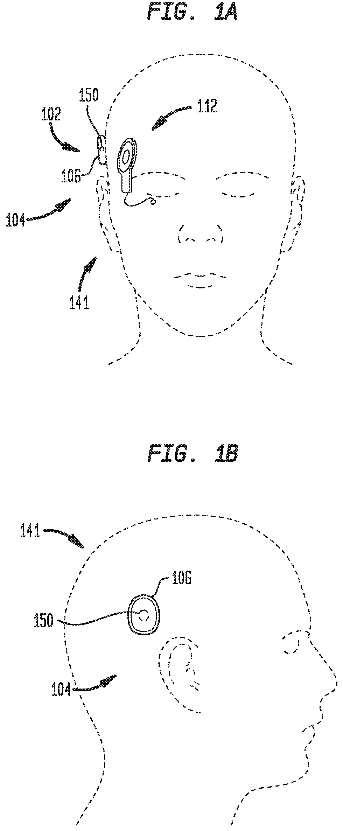

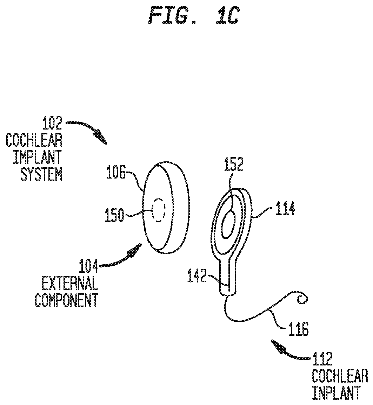

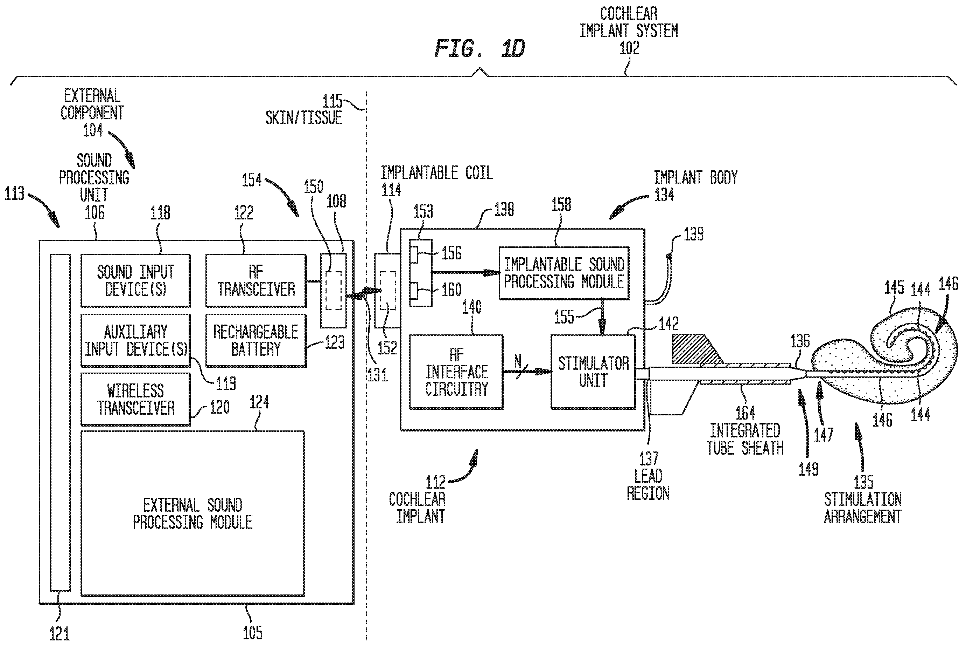

FIGs. 1A-1D illustrates an example cochlear implant system 102 with which certain embodiments of the techniques presented herein can be implemented. The cochlear implant system 102 comprises an external component 104 and an implantable component 112. In the examples of FIGs. 1A-1D, the implantable component is sometimes referred to as a "cochlear implant." FIG. 1A illustrates the cochlear implant 112 implanted in the head 141 of a recipient, while FIG. 1B is a schematic drawing of the external component 104 worn on the head 141 of the recipient. FIG. 1C is another schematic view of the cochlear implant system 102, while FIG. 1D illustrates further details of the cochlear implant system 102. For ease of description, FIGs. 1A-1D will generally be described together.

As noted, cochlear implant system 102 includes an external component 104 that is configured to be directly or indirectly attached to the body of the recipient and an implantable component 112 configured to be implanted in the recipient. In the examples of FIGs. 1A-1D, the external component 104 comprises a sound processing unit 106, while the cochlear implant 112 includes an internal coil 114, an implant body 134, and an elongate stimulation assembly 116.

In the example of FIGs. 1A-1D, the sound processing unit 106 is an off-the-ear (OTE) sound processing unit, sometimes referred to herein as an OTE component, that is configured to send data and power to the implantable component 112. In general, an OTE sound processing unit is a component having a generally cylindrically shaped housing 105 and which is configured to be magnetically coupled to the recipient's head (e.g., includes an integrated external magnet 150 configured to be magnetically coupled to an implantable magnet 152 in the implantable component 112). The OTE sound processing unit 106 also includes an integrated external (headpiece) coil 108 that is configured to be inductively coupled to the implantable coil 114.

It is to be appreciated that the OTE sound processing unit 106 is merely illustrative of the external devices that could operate with implantable component 112. For example, in alternative examples, the external component may comprise a behind-the-ear (BTE) sound processing unit or a micro-BTE sound processing unit and a separate external. In general, a BTE sound processing unit comprises a housing that is shaped to be worn on the outer ear of the recipient and is connected to the separate external coil assembly via a cable, where the external coil assembly is configured to be magnetically and inductively coupled to the implantable coil 114. It is also to be appreciated that alternative external components could be located in the recipient's ear canal, worn on the body, *etc.*

As noted above, the cochlear implant system 102 includes the sound processing unit 106 and the cochlear implant 112. However, as described further below, the cochlear implant 112 can operate with the sound processing unit 106 stimulate the recipient or the cochlear implant 112 can operate independently from the sound processing unit 106, for at least a period, to stimulate the recipient. For example, the cochlear implant 112 can operate in a first general mode, sometimes referred to as an "external hearing mode," in which the sound processing unit 106 captures sound signals which are then used as the basis for delivering stimulation signals to the recipient. The cochlear implant 112 can also operate in a second general mode, sometimes referred as an "invisible hearing" mode, in which the sound processing unit 106 is unable to provide sound signals to the cochlear implant 112 (e.g., the sound processing unit 106 is not present, the sound processing unit 106 is powered-off, the sound processing unit 106 is malfunctioning, etc.). As such, in the invisible hearing mode, the cochlear implant 112 captures sound signals itself via implantable sound sensors and then uses those sound signals as the basis for delivering stimulation signals to the recipient. Further details regarding operation of the cochlear implant 112 in the external hearing mode are provided below, followed by details regarding operation of the cochlear implant 112 in the invisible hearing mode. It is to be appreciated that reference to the external hearing mode and the invisible hearing mode is merely illustrative and that the cochlear implant 112 could also operate in alternative modes.

Referring first to the external hearing mode, FIGs. 1A-1D illustrate that the OTE sound processing unit 106 comprises one or more input devices 113 that are configured to receive input signals (e.g., sound or data signals). The one or more input devices 113 include one or more sound input devices 118 (e.g., one or more external microphones, audio input ports, telecoils, *etc*.), one or more auxiliary input devices 119 (e.g., audio ports, such as a Direct Audio Input (DAI), data ports, such as a Universal Serial Bus (USB) port, cable port, *etc*.), and a wireless transmitter/receiver (transceiver) 120. However, it is to be appreciated that one or more input devices 113 may include additional types of input devices and/or less input devices (e.g., the wireless short range radio transceiver 120 and/or one or more auxiliary input devices 119 could be omitted).

The OTE sound processing unit 106 also comprises the external coil 108, a charging coil 121, a closely-coupled transmitter/receiver (RF transceiver) 122, sometimes referred to as or radio-frequency (RF) transceiver 122, at least one rechargeable battery 123, and an external sound processing module 124. The external sound processing module 124 may comprise, for example, one or more processors and a memory device (memory) that includes sound processing logic. The memory device may comprise any one or more of: Non-Volatile Memory (NVM), Ferroelectric Random Access Memory (FRAM), read only memory (ROM), random access memory (RAM), magnetic disk storage media devices, optical storage media devices, flash memory devices, electrical, optical, or other physical/tangible memory storage devices. The one or more processors are, for example, microprocessors or microcontrollers that execute instructions for the sound processing logic stored in memory device.

The implantable component 112 comprises an implant body (main module) 134 and a stimulation arrangement 135, all configured to be implanted under the skin/tissue (tissue) 115 of the recipient. The implant body 134 generally comprises a hermetically-sealed housing 138 in which RF interface circuitry 140 and a stimulator unit 142 are disposed. The implant body 134 also includes the internal/implantable coil 114 that is generally external to the housing 138, but which is connected to the RF interface circuitry 140 via a hermetic feedthrough (not shown in FIG. 1D).

The stimulation arrangement 135 is described as comprising three (3) parts, namely the intra-cochlear stimulation assembly 116, a transition region 136, and a lead region 137. The stimulation assembly 116 is configured to be at least partially implanted in the recipient's cochlea 145 and includes a plurality of longitudinally spaced intra-cochlear electrical stimulating contacts (electrodes) 144 that collectively form a contact or electrode array 146 for delivery of electrical stimulation (current) to the recipient's cochlea 145.

Stimulation assembly 116 extends through an opening 147 in the recipient's cochlea 145 (e.g., cochleostomy, the round window, *etc*.) and has a proximal end connected to the transition region 136, which in turn is connected to the stimulator unit 142 via lead region 137 and a hermetic feedthrough (not shown in FIG. 1D). A plurality of conductors (wires) extend through the transition region 136 and the lead region 137 to electrically couple the electrodes 144 to the stimulator unit 142. The implantable component 112 also includes an electrode outside of the cochlea 145, sometimes referred to as the extra-cochlear electrode (ECE) 139.

As noted, the cochlear implant system 102 includes the external coil 108 and the implantable coil 114. The external magnet 152 is fixed relative to the external coil 108 and the implantable magnet 152 is fixed relative to the implantable coil 114. The magnets fixed relative to the external coil 108 and the implantable coil 114 facilitate the operational alignment of the external coil 108 with the implantable coil 114. This operational alignment of the coils enables the external component 104 to transmit data and power to the implantable component 112 via a closely-coupled wireless RF link 131 formed between the external coil 108 with the implantable coil 114. In certain examples, the closely-coupled wireless link 131 is a radio frequency (RF) link. However, various other types of energy transfer, such as infrared (IR), electromagnetic, capacitive and inductive transfer, may be used to transfer the power and/or data from an external component to an implantable component and, as such, FIG. 1D illustrates only one example arrangement.

As noted above, sound processing unit 106 includes the external sound processing module 124. The external sound processing module 124 is configured to convert received input signals (received at one or more of the input devices 113) into output signals for use in stimulating a first ear of a recipient (i.e., the external sound processing module 124 is configured to perform sound processing on input signals received at the sound processing unit 106). Stated differently, the one or more processors in the external sound processing module 124 are configured to execute sound processing logic in memory to convert the received input signals into output signals that represent electrical stimulation for delivery to the recipient.

As noted, FIG. 1D illustrates an embodiment in which the external sound processing module 124 in the sound processing unit 106 generates the output signals. In an alternative embodiment, the sound processing unit 106 can send less processed information (e.g., audio data) to the implantable component 112 and the sound processing operations (e.g., conversion of sounds to output signals) can be performed by a processor within the implantable component 112.

Returning to the specific example of FIG. 1D, the output signals are provided to the RF transceiver 122, which transcutaneously transfers the output signals (e.g., in an encoded manner) to the implantable component 112 via external coil 108 and implantable coil 114. That is, the output signals are received at the RF interface circuitry 140 via implantable coil 114 and provided to the stimulator unit 142. The stimulator unit 142 is configured to utilize the output signals to generate electrical stimulation signals (e.g., current signals) for delivery to the recipient's cochlea. In this way, cochlear implant system 102 electrically stimulates the recipient's auditory nerve cells, bypassing absent or defective hair cells that normally transduce acoustic vibrations into neural activity, in a manner that causes the recipient to perceive one or more components of the received sound signals.

As detailed above, in the external hearing mode the cochlear implant 112 receives processed sound signals from the sound processing unit 106. However, in the invisible hearing mode, the cochlear implant 112 is configured to capture and process sound signals for use in electrically stimulating the recipient's auditory nerve cells. In particular, as shown in FIG. 1D, the cochlear implant 112 includes a plurality of implantable sensors 153 and an implantable sound processing module 158. Similar to the external sound processing module 124, the implantable sound processing module 158 may comprise, for example, one or more processors and a memory device (memory) that includes sound processing logic. The memory device may comprise any one or more of: Non-Volatile Memory (NVM), Ferroelectric Random Access Memory (FRAM), read only memory (ROM), random access memory (RAM), magnetic disk storage media devices, optical storage media devices, flash memory devices, electrical, optical, or other physical/tangible memory storage devices. The one or more processors are, for example, microprocessors or microcontrollers that execute instructions for the sound processing logic stored in memory device.

In the invisible hearing mode, the implantable sensors 153 are configured to detect/capture signals (e.g., acoustic sound signals, vibrations, etc.), which are provided to the implantable sound processing module 158. The implantable sound processing module 158 is configured to convert received input signals (received at one or more of the implantable sensors 153) into output signals for use in stimulating the first ear of a recipient (i.e., the processing module 158 is configured to perform sound processing operations). Stated differently, the one or more processors in implantable sound processing module 158 are configured to execute sound processing logic in memory to convert the received input signals into output signals 155 that are provided to the stimulator unit 142. The stimulator unit 142 is configured to utilize the output signals 155 to generate electrical stimulation signals (e.g., current signals) for delivery to the recipient's cochlea, thereby bypassing the absent or defective hair cells that normally transduce acoustic vibrations into neural activity.

It is to be appreciated that the above description of the so-called external hearing mode and the so-called invisible hearing mode are merely illustrative and that the cochlear implant system 102 could operate differently in different embodiments. For example, in one alternative implementation of the external hearing mode, the cochlear implant 112 could use signals captured by the sound input devices 118 and the implantable sensors 153 in generating stimulation signals for delivery to the recipient.

As noted above, the cochlear implant 112 comprises implantable sensors 153. In certain embodiments, the implantable sensors 153 comprise at least two sensors 156 and 160, where at least one of the sensors is designed to be more sensitive to bone-transmitted vibrations than it is to acoustic (air-borne) sound waves. In the illustrative embodiment of FIG. 1D, the implantable sensor 156 is an implantable "sound" sensor/transducer that is primarily configured to detect/receive external acoustic sounds (e.g., an implantable microphone), while the implantable sensor 160 is a "vibration" sensor that is primarily configured to detect/receive internal vibration signals, including body noises. (e.g., another implantable microphone or an accelerometer). These sensors can take a variety of different forms, such as another implantable microphone, an accelerometer, *etc.* However, for ease of description, embodiments presented herein will be primarily described with reference to the use of an implantable microphone as the sound sensor and an accelerometer as the vibration sensor. The increased sensitivity of the accelerometer to vibration signals (e.g., body noises) may be due to, for example, the structure of the accelerometer relative to the microphone, the implanted position of the accelerometer relative to the microphone, *etc.* For example, in certain embodiments, the accelerometer and the microphone are structurally similar but they are placed in different locations which accounts for the vibration/body noise sensitivity difference. Again, it is to be appreciated that these specific implementations are non-limiting and that embodiments of the present disclosure may be used with different types of implantable sensors

The implantable microphone 156 and the accelerometer 160 can each be disposed in, or electrically connected to, the implant body 134. In operation, the implantable microphone 156 and the accelerometer 160 each detect input signals and convert the detected input signals into electrical signals. The input signals detected by the implantable microphone 156 and the accelerometer 160 can each include external acoustic sounds and/or vibration signals, including body noises.

In the example of FIGs. 1A-1D, the stimulation assembly 116 is a pre-curved perimodiolar stimulation assembly configured to adopt a curved configuration during and/or after implantation into the cochlea 145. To achieve this, in certain embodiments, the perimodiolar stimulation assembly is pre-curved to the same general curvature of the cochlea 145, but is kept in a straight configuration during at least a portion of the implantation process. That is, the perimodiolar stimulation assembly 116 needs to be straightened in order to be inserted into the cochlea 145 via an opening in the cochlea (e.g., cochleostomy, oval window, etc.).

In the example of FIG. 1D, during insertion into the cochlea 145, the example perimodiolar stimulation assembly 116 is constrained (e.g., held) straight by an integrated tube sheath 164. That is, the integrated tube sheath 164 is configured to structurally support the stimulation assembly 116 during insertion of the stimulation assembly 116 into the cochlea 145. As described further below, the integrated tube sheath 164 is referred to as being "integrated" because it is configured to be permanently disposed around (e.g., is not removable from) the stimulation arrangement 135. That is, as described further below, the integrated tube sheath 164 remains implanted in the recipient after insertion of the stimulation assembly 116, for the lifetime of the stimulation arrangement 135.

The integrated tube sheath 164 is, as suggested by the name, has a "complete" or "full" elongate tubular shape. That is, the integrated tube sheath 164 is configured to extend completely around an outer circumference 149 of the stimulation arrangement 135. FIG. 1D illustrates an embodiment in which the integrated tube sheath 164 is initially disposed around the stimulation assembly 116 during insertion of the stimulation assembly into the cochlea 145. However, in this example, the integrated tube sheath 164 is configured to be removed from the cochlea 145, but is configured to be positioned on, and to remain on, the transition region 136, following insertion of the stimulation assembly 116 into the cochlea 145. However, in other embodiments, an integrated tube sheath in accordance with embodiments presented herein can remain partially within the cochlea 145 following insertion of the stimulation assembly 116 into the cochlea 145.

Since the integrated tube sheaths presented herein are configured to remain implanted in the recipient, the integrated tubular sheaths presented advantages do not a longitudinal slit extending along the length thereof. The lack of longitudinal slit allows the integrated tube sheaths to be made of a softer material (for example silicone, instead of polyimide) without risk of the stimulating assembly coming out the side of the sheath via such a slit. A softer sheath has the potential to be less traumatic and better able to preserve structure and acoustic hearing function. That is, the presence of longitudinal slits presents a challenge as the sheath needs to be simultaneously very flexible in terms of bending, to minimize insertion trauma, while having enough stiffness as a tube to straighten the stimulation assembly and keep it from coming out through the slit during insertion.

Although FIG. 1D illustrates use of the integrated tube sheath 164 with the pre-curved stimulation assembly 116, it is to be appreciated that this embodiment is merely illustrative and that integrated tube sheaths described herein can be used with a variety of different types of stimulation assemblies (e.g., straight; curved; elongated; short) configured to be implanted in different areas/cavities of a recipient's body (e.g., cochlea, vestibular system, brain stem, etc.). However, merely for ease of description, the integrated tube sheaths presented herein will primarily be described with reference to pre-curved perimodiolar stimulation assemblies configured to be implanted in a recipient's cochlea.

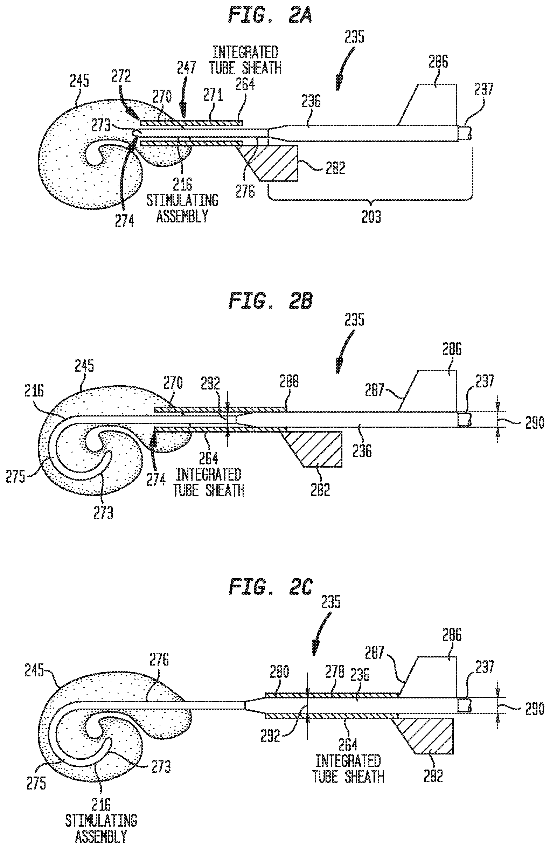

FIGs. 2A-2E are a series of schematic diagrams illustrating cross-sectional views of an example integrated tube sheath 264, in accordance with certain embodiments presented herein. In these examples, the integrated tube sheath 264 is shown integrated with (e.g., not removable from) a stimulation arrangement 235 configured to be partially implanted in a recipient's cochlea 245. For ease of illustration, FIGs. 2A-2E illustrate cross-sectional views of the integrated tube sheath 264. However, as described further below, the integrated tube sheath 264 is a complete elongate tubular structure (tube) that fully surrounds/encloses an elongate length of the stimulation arrangement 235.

The stimulation arrangement 235 comprises a pre-curved perimodiolar intra-cochlear stimulation assembly 216 forming a distal portion of the stimulation arrangement 235, a transition region 236 connected to a proximal end of the stimulation assembly, and a lead region 237, connected to a proximal end of the transition region 236. Presence of both the lead region 237 and the transition region 236 is merely illustrative and, in certain embodiments, the lead region 237 and/or the transition region 236 may be omitted and/or these regions can be formed as a single region. For ease of description, the lead region 237 and/or the transition region 236 are collectively and generally referred to as a "transition region" 203 (FIG. 2A) connected to a proximal end of the stimulation assembly 216 (e.g., the stimulation arrangement 235 generally comprises the stimulation assembly 216 and a transition region 203).

In the examples of FIGs. 2A-2E, the integrated tube sheath 264 is a complete tube sheath that is slideably moveable along the stimulation arrangement 235, but is not removable therefrom. That is, the tube sheath 264 stays on the stimulation arrangement 235 in the body of the recipient for the life of the stimulation arrangement. The integrated tube sheath 264 is further configured to provide sufficient rigidity to maintain the pre-curved perimodiolar stimulation assembly 216 in a substantially straight configuration during at least a portion of the implantation process.

In the examples of FIGs. 2A-2E, a proximal end of the cochlea region 203 (e.g., a proximal end of the lead region) is mechanically and electrically connected to an implant body, such as implant body 134 of FIG. 1D, that houses the electrically circuity configured to, for example, stimulate the recipient via the stimulation assembly 216. The implant body has dimensions that are significantly larger than those of the stimulation arrangement 235 such that the implant body physically blocks proximal motion of the integrated tube sheath 264 (e.g., prevents proximal motion of the integrated tube sheath 264 beyond the proximal end of the transition region 203). Therefore, as described further below, the integrated tube sheath 264 has a configuration such that the integrated tube sheath 264 can remain permanently within the body of the recipient (e.g., for the lifetime of the stimulation arrangement 235), without negative consequences, such as biofilm formation between the integrated tube sheath 264 and the stimulation arrangement 235, fluidically sealing the cochlea 245, etc.

In certain embodiments, the implantation process for stimulation assembly 216 includes creating a surgical opening (e.g., facial recess) in the body of the recipient, such as through the recipient's mastoid bone to access the recipient's middle ear cavity. The surgical process further includes forming or accessing an opening 247 (e.g., cochleostomy, round window, oval window, etc.) in the cochlea 245. The stimulation assembly 216 is initially positioned in (e.g., encased in and surrounded by) the integrated tube sheath 264, and is inserted through the surgical opening to the opening 247 in the cochlea 245. That is, the stimulation assembly 216 and the surrounding tube sheath 264 are advanced (e.g., pushed) through the opening in the mastoid bone and are positioned to be inserted into the opening 247. In this configuration, the integrated tube sheath 264 provides the rigidity to maintain the pre-curved perimodiolar stimulation assembly 216 in the substantially straight configuration.

As schematically illustrated in FIG. 2A, the stimulation assembly 216 and the integrated tube sheath 264 are advanced (e.g., pushed) together through the opening 247 to insert a distal portion 270 of the sheath 264 into the cochlea 245 while the stimulation assembly 216 remains in the sheath. Stated differently, a distal end 273 of the stimulation assembly 216 and a distal end 272 of the elongate tube sheath 264 (since the sheath is disposed around the stimulation assembly) are positioned within the cochlea 245. The advancement of the stimulation assembly 216 and the surrounding integrated tube sheath 264 into the cochlea 245 is stopped once the distal end 272 of the sheath 264 is in a predetermined position (e.g., a certain distance inside cochlea 245).

As schematically illustrated by FIG. 2B, the stimulation assembly 216 is then gently advanced (e.g., pushed) forward into the cochlea 245 through the integrated tube sheath 264 (e.g., a distal end 273 of the stimulation assembly 216 exits the sheath 264 through an opening 274 in the distal end 272 of the integrated tube sheath 264). That is, the stimulation assembly 216 slides relative to (through) the integrated tube sheath 264. The pre-curved distal portion 275 of the stimulation assembly 216 that extends out of the integrated tube sheath 264 is no longer constrained to be straight by the integrated tube sheath 264 and therefore returns to its pre-curved configuration to follow the curvature of the canals within the cochlea 245. As schematically illustrated by FIG. 2B, the advancement of the stimulation assembly 216 continues until the stimulation assembly 216 achieves the implanted position (e.g., the distal end 273 of the stimulation assembly 216 is at or adjacent the cochlea apex). For example, the implanted position of the stimulation assembly 216 can be the position at which the distal end 273 of the stimulation assembly 216 is placed at a selected angular position (e.g., angular insertion depth; angular rotation of the distal end 273 of the stimulation assembly 216 from the opening 247 through which the stimulation assembly 216 enters the cochlea 245).

As shown in FIG. 2C, once the stimulation assembly 216 achieves the implanted position, the distal end 272 of the integrated tube sheath 264 can be withdrawn from the cochlea 245 (e.g., pulled out) through the opening 247. In a final resting position shown in FIG. 2C, the integrated tube sheath 264 is disposed around, and generally mates with, the transition region 236. That is, in this final resting position, an inner surface 278 of the tube sheath 264 is positioned abutting (e.g., in contact with) an outer surface 280 of the transition region 236 so as to inhibit the formation of biofilm there between.

In accordance with certain examples presented herein, the integrated tube sheath 264 is a complete/full tube sheath that is slideably moveable along the stimulation arrangement 235, but is not removable (e.g., an inner surface of the tube sheath is configured to slideably engage an outer surface of the stimulation arrangement 235, such as an outer surface of the transition region 236). In certain examples, the stimulation assembly 216 is packaged with the sheath 264 on the transition region 236 and a basal portion 276 of stimulation assembly 216, with the pre-curved distal portion 275 of the stimulation assembly 216 extending out from the opening 274. In such embodiments, the surgeons pulls the stimulation assembly 216 backwards such that the pre-curved distal portion 275 is pulled into the tube sheath 264 and thereby held straight for insertion.

Thereafter, as noted above, the surgeon inserts the integrated tube sheath a short distance into the cochlea 245, then advances the stimulation assembly 216 out of the integrated tube sheath to its final position. The integrated tube sheath 264 is then slid in a proximal direction (along the stimulation arrangement 235 the implant body) so that no part of the integrated tube sheath 264 remains in the cochlea 245. Also as noted, the integrated tube sheath 264 is slid to a final resting position on the transition region 236 (e.g., between the stimulation assembly 216 and the implant body), where the integrated tube sheath 264 remains for the implanted life of the device.

In the example of FIGs. 2A-2E, the integrated tube sheath 264 includes a handle 282, while the stimulation arrangement 235 also includes a handle 286 proximal to the sheath 264 (e.g., the handle is located at a proximal end of the transition region 236). The final resting position for the integrated tube sheath 264 may be, in certain examples, where a distal edge 287 of the handle 286 contacts a proximal edge 288 of the sheath.

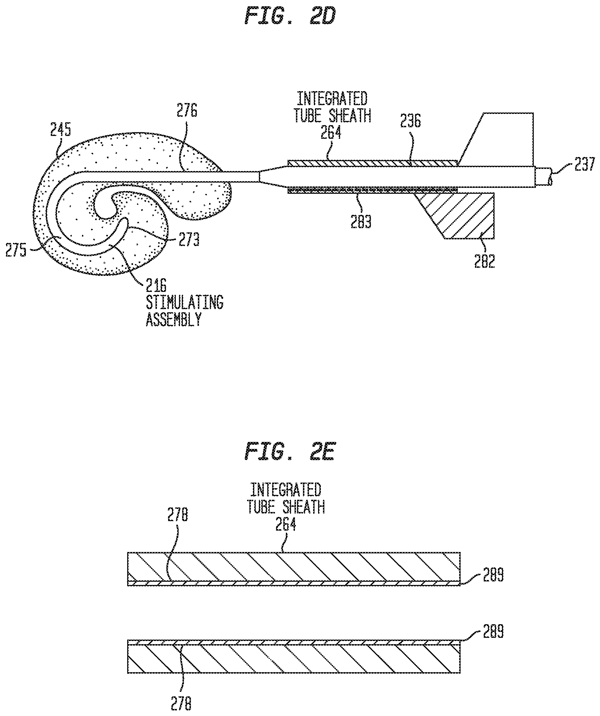

In the example of FIGs. 2A-2E, the transition region 236 has a stiffness/rigidity (e.g., between the handle 286 and the stimulation assembly 216) to enable advancement of the stimulation assembly 216 against some resistance without buckling. Similarly, the integrated tube sheath 264 must be stiff enough (at least in the proximal region 271 that does not enter the cochlea 245) to prevent buckling. In certain embodiments, the integrated tube sheath 264 may be constructed with an embedded stiffener that extends from the handle 282 through the proximal region 271 or just beyond. The stiffener may be malleable, to allow it to be formed to a desired shape and retain that form, preventing the 'springiness' of the lead from moving the stimulation assembly 216 after insertion. FIG. 2D illustrates an example in which an elongate stiffener 283 is disposed in the integrated tube sheath 264.

In certain embodiments, since the integrated tube sheath 264 remains implanted in the recipient, the integrated tube sheath 264 may have a fixation feature, such as a silicone or metal-in-silicone protrusion, that could be secured (e.g., screwed or glued) to the bone in the mastoid. In certain embodiments, the handle 282 may operate as a fixation feature that can be secured to the recipient's bone. The integrated tube sheath 264 can also have a longitudinal stiffness configured to prevent the stimulation assembly 216 from backing out of the cochlea 245.

As noted, the integrated tube sheath 264 remains in the body of the recipient and, in the final resting position, abuts the transition region 236. As noted above, in order to prevent biofilm formation between the transition region 236 and the integrated tube sheath 264, the integrated tube sheath 264 is configured to achieve a tight fit on (e.g., mate with) the transition region 236 in the final resting position to prevent/inhibit biofilm formation. This may be achieved, for example, by forming the transition region 236 with an outer diameter 290 that is slightly larger than an inner diameter 292 of the integrated tube sheath 264. Therefore, in such an embodiment, the transition region 236 is configured to apply an outward force on the integrated tube sheath 264 to ensure no gap is present between the inner surface 278 of the tube sheath 264 and the outer surface 280 of the transition region 236. In further embodiments, antimicrobial and/or antifouling substances could also be added to the inner surface 278 of the tube sheath 264 and/or the outer surface 280 of the transition region 236. FIG. 2E schematically illustrates antimicrobial substance 289 disposed on the inner surface 278 of the tube sheath 264. Example antimicrobial substances that can be used in accordance with embodiments present herein can include coatings comprise of silver oxide, silver alloys or silver nanoparticles, polymeric substances such as zwitterionic coatings, etc.

In summary, FIGs. 2A-2E illustrate an integrated tube sheath 264 that is removed from the cochlea 245 following insertion of the stimulation assembly 216. However, the integrated tube sheath 264 has a solid/complete tube shape that cannot be removed from the stimulation arrangement 235 (e.g., a proximal end of the stimulation arrangement 235 is connected to the implant body (not shown), thereby physically preventing further proximal movement of the tubular sheath).

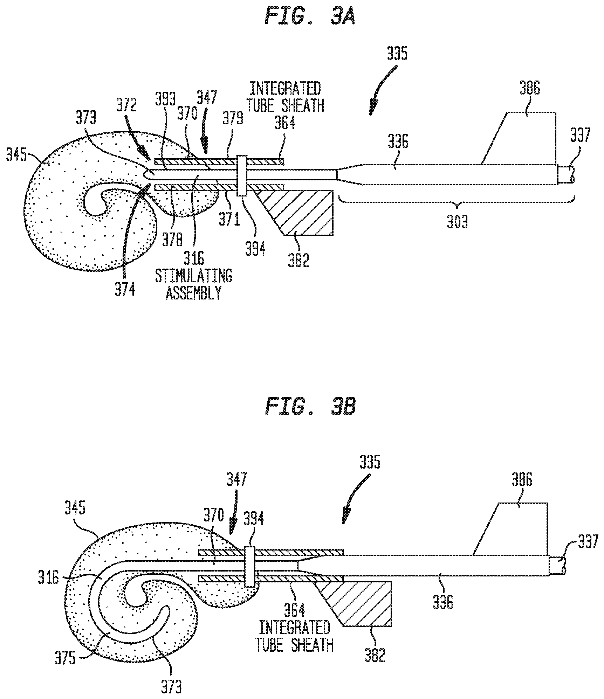

FIGs. 3A and 3B illustrate are a series of schematic diagrams illustrating aspects of another example integrated tube sheath 364, in accordance with embodiments presented herein. In these examples, the integrated tube sheath 364 is shown integrated with (e.g., permanently disposed around and not removable from) a stimulation arrangement 335 configured to be partially implanted in a recipient's cochlea 345. The stimulation arrangement 335 comprises a pre-curved perimodiolar intra-cochlear stimulation assembly 316, a transition region 336, and a lead region 337. Again, the transition region 336 and the lead region 337 are illustrative and are collectively and generally referred to as transition region 303 (FIG. 3A). For ease of illustration, FIGs. 3A and 3B illustrate cross-sectional views of the integrated tube sheath 364. However, as described further below, the integrated tube sheath 364 is a complete elongate tubular structure (tube) that fully surrounds an outer circumference of the stimulation arrangement 335 for an elongate length thereof.

In the examples of FIGs. 3A and 3B, the integrated tube sheath 364 is slideably moveable along the stimulation arrangement 335, but is not removable therefrom (e.g., the sheath 364 remains permanently on the stimulation arrangement 335 in the body of the recipient for the life of the stimulation arrangement). The stimulation assembly 316 is initially positioned in (e.g., encased in and surrounded by) the integrated tube sheath 364, and the stimulation assembly 316 and the surrounding sheath 364 are advanced (e.g., pushed) through the opening through the mastoid bone and are positioned to be inserted into the opening 347. In this configuration, the integrated tube sheath 364 provides the structural support (rigidity) to maintain the pre-curved perimodiolar stimulation assembly 316 in the substantially straight configuration.

As schematically illustrated in FIG. 3A, the stimulation assembly 316 and the integrated tube sheath 364 are advanced (e.g., pushed) together through the opening 347 to insert a distal portion 370 of the tube sheath 364 into the cochlea 345 while the stimulation assembly 316 remains in the sheath. The advancement of the stimulation assembly 316 and the surrounding integrated tube sheath 364 is stopped once a distal end 372 of the sheath 364 is in a predetermined position (e.g., a certain distance inside cochlea 345).

As schematically illustrated by FIG. 3B, the stimulation assembly 316 is then gently advanced (e.g., pushed) forward into the cochlea 345 through the integrated tube sheath 364 (e.g., a distal end 373 of the stimulation assembly 316 exits the sheath 364 through an opening 374 in the distal end 372 of the integrated tube sheath 364). The pre-curved distal portion 375 of the stimulation assembly 316 that extends out of the integrated tube sheath 364 is no longer constrained to be straight by the integrated tube sheath 364 and therefore returns to its pre-curved configuration to follow the curvature of the canals within the cochlea 345. As schematically illustrated by FIG. 3B, the advancement of the stimulation assembly 316 continues until the stimulation assembly 316 achieves the implanted position (e.g., the distal end 373 of the stimulation assembly 316 is at or adjacent the cochlea apex. For example, the implanted position can be the position at which the distal end 373 of the stimulation assembly 316 is placed at a selected angular position (e.g., angular insertion depth; angular rotation of the distal end 373 of the stimulation assembly 316 from the opening 347 through which the stimulation assembly 316 enters the cochlea 345).

As shown in FIG. 3B, once the stimulation assembly 316 achieves the implanted position, the distal portion 370 of integrated tube sheath 364 is configured to remain permanently (e.g., for the lifetime of device) within the cochlea 345. That is, in the embodiments of FIGs. 3A and 3B, the final resting position for the integrated tube sheath 364 includes the distal portion 370 located within the cochlea 345, and a proximal portion 371 located outside of the cochlea (e.g., the integrated tube sheath 364 is partially into the cochlea 345 to deliver the stimulation assembly 316, and then remains in this position for the implanted life of the device). In comparison to the embodiments of FIGs. 2A-2E, the embodiments of FIGs. 3A and 2B do not include the final step of withdrawing the tube sheath out from the cochlea, which has the advantages of a simpler surgical process, as well as allowing the sheath handle 382 to be closer to the proximal end of the tube and therefore reducing the buckling risk during the insertion.

In certain examples, the distal portion 370 of the integrated tube sheath 364 will cover the basal most electrodes/contacts on the stimulation assembly 316. As such, the distal portion 370 of the integrated tube sheath 364 is configured to allow stimulation current to flow from the electrodes to the recipient's tissue. In certain embodiments, the distal portion 370 of the integrated tube sheath 364 can have a perforated or mesh-type construction (e.g., electrically-transparent structure). Alternatively, the distal portion 370 of the integrated tube sheath 364 can be partially or fully bioresorable so as to dissolve, in situ, over a period of time (e.g., several minutes, several hours, several days, etc.).

As noted above, the integrated tube sheath 364 extends through the opening 347 and could provide a pathway between the inner ear and middle ear that might allow perilymph to leak out of the cochlea 345 and/or for pathogens to track into (enter) the cochlea. As such, the integrated tube sheath 364 is configured to fluidically seal the opening 347, both inside and around the outer circumference (outside) of the sheath. In the example of FIGs. 3A and 3B, the seal is provided by slideable sealing member 394. In operation, the slideable sealing member 394 is slideable along (relative to) both the inner surface 378 and the integrated tube sheath 364, the outer surface 379 of the integrated tube sheath 364, and the outer surface 393 of the stimulation assembly 316.

During insertion of the stimulation assembly 316 into the cochlea 345, the slideable sealing member 394 has a proximal position adjacent the handle 382. However, after insertion of the stimulation assembly 316 into the cochlea 345, the surgeon can move (slide) the slideable sealing member 394 in a distal direction so as to be positioned abutting the opening 347. This position, which is shown in FIG. 3B, enables the slideable sealing member 394 to seal the opening 394. In certain embodiments, the slideable sealing member 394 is configured to form a seal around the outer surface 379 of the integrated tube sheath 364 by, for example, applying an inward compressive force on the outer surface of the sheath. Application of this inward compressive force on the outer surface 379 of the integrated tube sheath 364 forms a fluidic seal between the slideable sealing member 394 and the outer surface integrated tube sheath 364. The slideable sealing member 394 can be secured to (e.g., via an adhesive) to the cochlea 345 around the opening 347.

Similarly, the slideable sealing member 394 is configured to fill the interior of the integrated tube sheath 364 between the inner surface 378 of the sheath and the outer surface 393 of the stimulation assembly 316. For example, the slideable sealing member 394 may be configured (e.g., shaped, have material properties, etc.) to apply an inward compressive force on the outer surface of the stimulation assembly 316 to form a fluidic seal between the integrated tube sheath 364 and the stimulation assembly 316. In certain embodiments, the inward compressive force applied on the outer surface 379 of the integrated tube sheath 364 is sufficient to also form a fluidic seal between the slideable sealing member 394 and the inner surface 378 of the sheath. In certain embodiments, the slideable sealing member 394 may be configured (e.g., shaped, have material properties, etc.) to apply an outward compressive force on the inner surface 378 of the stimulation assembly 316 to form a fluidic seal between the inner surface of the integrated tube sheath 364 and the slideable sealing member 394.

In alternative embodiments, the slideable sealing member 394 of FIGs. 3A and 3B could be replaced by, of used in combination with, a fixed section of the integrated tube sheath 364 in which the inner diameter of the sheath is slightly less than the outer diameter of the stimulation assembly 316, providing an interference fit at the opening 347. The fixed section could be also or alternatively be achieved by providing one or more ribs on the inner surface 378 of the integrated tube sheath 364 and/or one or more ribs on the outer surface 393 of the stimulation assembly 316.

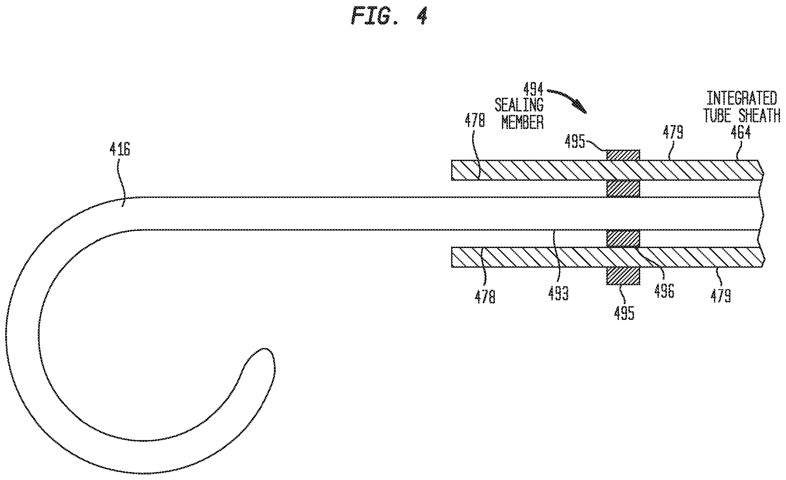

For example, FIG. 4 is a cross-sectional view of an integrated tube sheath 464 having a fixed sealing member 494, in accordance with embodiments presented herein. Similar to the embodiments of FIGs. 3A and 3B, the integrated tube sheath 464 is configured to be inserted into, and remain within, the cochlea of a recipient. For ease of description, the integrated tube sheath 464 is shown with a stimulation assembly 416, but without the cochlea into which the integrated tube sheath 464 and stimulation assembly 416 are inserted.

In the example of FIG. 4, the fixed sealing member 494 comprises an outer annular member 495 (ring) extending about the circumference of the outer surface 479 of the integrated tube sheath 464. When the integrated tube sheath 464 is inserted into the cochlea (not shown), the outer annular member 495 is configured to function as a stopper to prevent over-insertion of the sheath into the cochlea. That is, the outer annular member 495 has a configuration (e.g., size, shape, material properties, etc.) such that the outer annular member 495 cannot pass through the opening in the cochlea. After the stimulation assembly 416 is fully inserted, the outer annular member 495 can be secured (e.g., adhered) to the cochlea to fluidically seal the cochlea opening through which the integrated tube sheath 464 extends.

The sealing member 494 also comprises an inner annular member 496 extending about the circumference of the inner surface 478 of the integrated tube sheath 464. The inner annular member 496 has an inner diameter that is slightly less than the outer diameter of the stimulation assembly so as to provide an interference fit with the stimulation assembly. The stimulation assembly 416 can move longitudinally relative to the inner annular member 496, but the inner annular member 496 forms a fluidic seal with the outer surface 493 of the stimulation assembly 416 when the stimulation assembly 416 is stationary (e.g., after insertion into the cochlea).

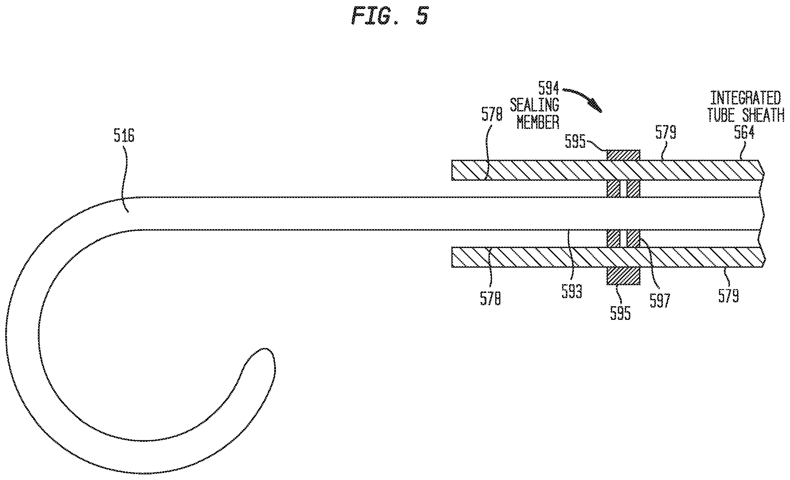

FIG. 4 illustrates an embodiment that comprises one inner annual ring 496. However, it is to be appreciated that alternative embodiments can comprise two or more inner annual rings. In addition, the inner annual rings can alternatively be formed on the stimulation assembly. FIG. 5 illustrates an example of such an embodiment.

More specifically, the embodiment of FIG. 5 is similar to the embodiment of FIG. 4 where an integrated tube sheath 564 having comprises a fixed sealing member 594. Similar to the embodiments of FIGs. 3A and 3B, the integrated tube sheath 564 is configured to be inserted into, and remain within, the cochlea of a recipient. For ease of description, the integrated tube sheath 564 is shown with a stimulation assembly 516, but without the cochlea into which the integrated tube sheath 564 and stimulation assembly 516 are inserted.

In the example of FIG. 4, the fixed sealing member 594 comprises an outer annular member 595 (ring) extending about the circumference of the outer surface 579 of the integrated tube sheath 564. When the integrated tube sheath 564 is inserted into the cochlea (not shown), the outer annular member 595 is configured to function as a stopper to prevent over-insertion of the sheath into the cochlea. After the stimulation assembly 516 is fully inserted, the outer annular member 595 can be secured (e.g., adhered) to the cochlea to seal the cochlea opening through which the integrated tube sheath 564 extends.

The sealing member 594 also comprises a plurality of annular members 597 extending about the circumference of the outer surface 593 of the stimulation assembly 516. The outer diameter annular members 597 are slightly greater than the inner diameter of the integrated tube sheath 564 so as to provide an interference fit with the sheath. The stimulation assembly 516 can move longitudinally relative to inner surface 578 of the integrated tube sheath, but the plurality of annular members 597 form a fluidic seal with the inner surface 578 of the integrated tube sheath when the stimulation assembly 416 is stationary (e.g., after insertion into the cochlea).

Alternatively, the seal between the stimulation assembly 616 and the sheath 664 can be made by a section of the stimulation assembly 616 that swells after insertion, for example using a hydrogel outer layer or a hydrogel core. An equivalent feature could be used to form a seal between the sheath and the cochlear opening.

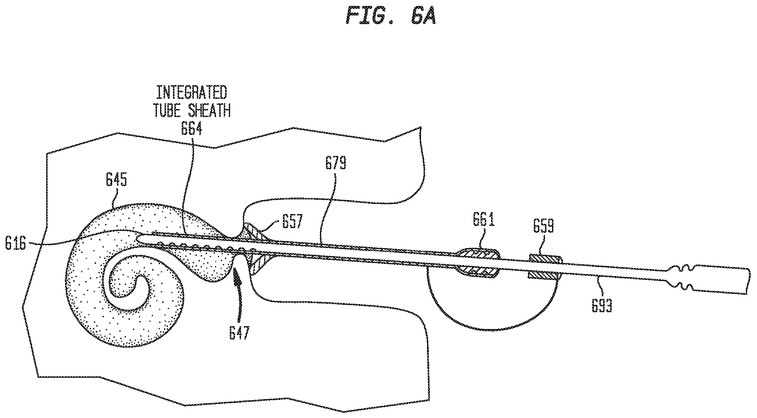

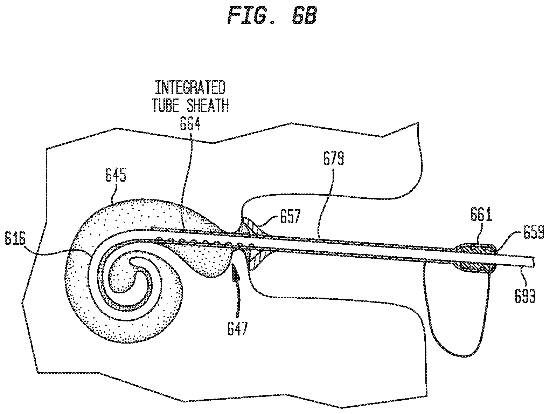

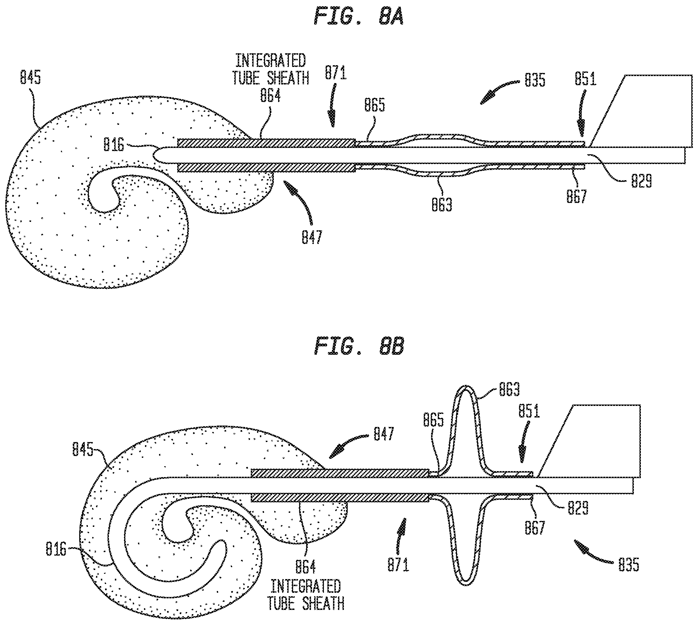

FIGs. 6A and 6B illustrate another embodiment of an integrated tube sheath 664 configured to remain partially positioned in a cochlea 645 of a recipient, in accordance with certain embodiments presented herein. The integrated tube sheath 664 operates similar to the above embodiments of, for example, FIGs. 3A-3C, to retain a pre-curved stimulation assembly 616 in a substantially straight configuration during insertion of the stimulation assembly 616 into the cochlea 645. FIG. 8A illustrates the arrangement of the integrated tube sheath 664 and stimulation assembly 616 during insertion, while FIG. 8B illustrates the arrangement of the integrated tube sheath 664 and stimulation assembly 616 following insertion.

In the examples of FIGs. 6A and 6B, the integrated tube sheath 664 is configured to, following insertion of the stimulation assembly 616 and the integrated tube sheath 664, fluidically seal the cochlea 645 via a combination of an annular stopper 657 and a plug 659. More specifically, the annular stopper 657 comprises an annular or ring member extending about the circumference of the outer surface 679 of the integrated tube sheath 664. When the integrated tube sheath 664 is inserted into the cochlea 645, the annular stopper 657 is configured to prevent over-insertion of the sheath into the cochlea. That is, the annular stopper 657 has a configuration (e.g., size, shape, material properties, etc.) such that the annular stopper 657 cannot pass through the opening 647 in the cochlea 645. As shown, after the stimulation assembly 616 is fully inserted into the cochlea 645, the annular stopper 657 abuts the opening 647. The annular stopper 657 can then be secured (e.g., adhered) to the cochlea to fluidically seal the cochlea opening 647 around the integrated tube sheath 664.

In the example of FIGs. 6A and 6B, the annular stopper 657 only fluidically seals the cochlea opening 647 around the outer surface 679 of the integrated tube sheath 664 and, as such, there remains a fluidic pathway within the interior of the sheath. As such, the integrated tube sheath 664 comprises the plug 659 that is configured to mate with a plug port 661 located at the proximal end of the integrated tube sheath 664 to fluidically seal the proximal end of the sheath.

The plug 659 comprises an annular (ring-shaped) member that is disposed around a proximal portion of the stimulation assembly 616. The plug 659 is slideable relative to the stimulation assembly 616 (e.g., is configured to slide along the outer surface 693 of the stimulation assembly 616) and to be inserted into the plug port 661 and/or the plug 659 may be integral with the stimulation assembly (as in FIG. 6B, thus creating a seal with port 661 only at a predefined insertion depth). When inserted into the plug port 661, the plug 659 is configured to fill the interior of the plug port and can be configured (e.g., shaped, have material properties, etc.) to apply an outward compressive force to form a fluidic seal with the plug port. Similarly, the plug 659 is configured to apply an inward compressive force to the outer surface 693 of the stimulation assembly 616 that is sufficient to also form a fluidic seal between the plug 659 and stimulation assembly 616. Stated differently, the plug 659 and plug port 661, as well as the plug 659 and the outer surface 693 of the stimulation assembly 616, are configured for an interference fit to form the fluidic seals. Although FIGs. 6A and 6B illustrate embodiments that utilize an interference fit, it is to be appreciated that other mechanisms (e.g., corresponding screw threads) could also or alternatively be used to form one or more of the fluidic seals.

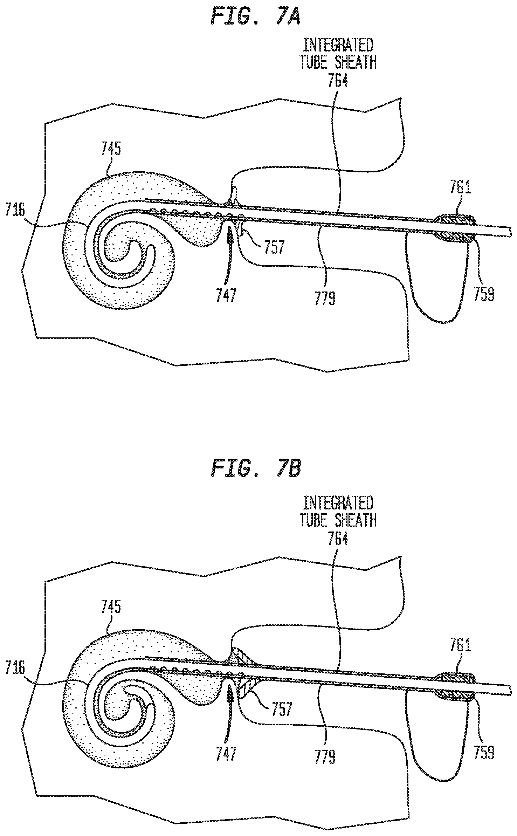

FIGs. 7A and 7B illustrate an integrated tube sheath 764 that is similar to the integrated tube sheath 664 of FIGs. 6A and 6B in that tube sheath 764 includes a plug 759 configured to mate with a plug port 761 at a proximal end of the sheath. The integrated tube sheath 764 also includes an annular stopper 757 comprising an annular or ring member extending about the circumference of the outer surface 779 of the integrated tube sheath 764. When the integrated tube sheath 764 is inserted into the cochlea 745, the annular stopper 757 is configured to prevent over-insertion of the sheath into the cochlea. That is, the annular stopper 757 has a configuration (e.g., size, shape, material properties, etc.) such that the annular stopper 757 cannot pass through the opening 747 in the cochlea 745. As shown, after the stimulation assembly 716 is fully inserted into the cochlea 745, the annular stopper 757 abuts the opening 747. The annular stopper 757 can then be secured (e.g., adhered) to the cochlea to fluidically seal the cochlea opening 747 around the integrated tube sheath 764.

In the examples of FIGs. 6A and 6B, the annular stopper 657 is substantially rigid. In contrast, the annular stopper 757 of FIGs. 7A and 7B is resiliently flexible (e.g., has springlike properties) that allow for slight over-insertion of the stimulation assembly 716 followed by a pull back. This over-insertion and pull-back can enable the stimulation assembly 716 to obtain an optimal position in the cochlea 745 that combines maximum insertion depth and close modiolar proximity. The annular stopper 757 may be designed as a conical flange (e.g., Belleville washer or disc spring) or may include a metal or plastic coil spring.

FIGs. 8A and 8B illustrate another embodiment of an integrated tube sheath 864 configured to remain partially positioned in a cochlea 845 of a recipient, in accordance with certain embodiments presented herein. The integrated tube sheath 864 operates similar to the above embodiments of, for example, FIGs. 3A-3C, to retain a pre-curved stimulation assembly 816 in a substantially straight configuration during insertion of the stimulation assembly 816 into the cochlea 845.

FIG. 8A illustrates the arrangement of the integrated tube sheath 864 and stimulation assembly 816 during insertion, while FIG. 8B illustrates the arrangement of the integrated tube sheath 864 and stimulation assembly 816 following insertion. The stimulation assembly 816 is a component of a stimulation arrangement 835.

In the examples of FIGs. 8A and 8B, the integrated tube sheath 864 is again configured to, following insertion of the stimulation assembly 816 and the integrated tube sheath 864, fluidically seal the cochlea 845. More specifically, FIGs. 8A and 8B illustrate that the stimulation arrangement 835 includes a proximally positioned flexible tube section 863 having a distal end 865 connected to a proximal section 871 of the integrated tube sheath 864, and a proximal end 867 connected to a proximal section 829 of the stimulation arrangement 835. The flexible tube section 863 is configured to extend a certain distance relative to the stimulation assembly 816, but maintains a continuous barrier to fluid and microbes. As such, fluid from the cochlea 845 can enter the interior of the integrated tube sheath 864, but cannot pass the point 851 at which the proximal end 867 of the flexible tube section 863 is connected to the proximal section 829 of the stimulation arrangement 835.

In certain embodiments, the flexible tube section 863 is a thin walled elastomer tube. To straighten the stimulation assembly 816 for insertion, the stimulation assembly 816 is pulled back into the integrated tube sheath 864, stretching the flexible tube section 863 to its extended configuration. To advance the stimulation assembly 816 into the cochlea 845, the stimulation assembly 816 is pushed out of the tube sheath 864, collapsing the flexible tube section 863 to its compressed configuration. The flexible tube section 863 may take the form of a concertina-shaped tube with multiple 'waves' or a single large 'wave.'

In the example of FIGs. 8A and 8B, the flexible tube section 863 seals the cochlea 845 with respect to the interior of the integrated tube sheath 864. It is to be appreciated that any of the sealing mechanisms described elsewhere herein (e.g., slideable member, rigid stopper, flexible stopper, hydrogel outer layer or a hydrogel, etc.) could be used to seal the cochlea 845 with respect to the exterior of the integrated tube sheath 864 (e.g., around the outer circumference of the tube sheath). In certain embodiments, the integrated tube sheath 864 may be self-sealing. That is, the integrated tube sheath 864 can be configured (e.g., size, shape, material properties, etc.) to form a direct fluidic seal with a portion of the cochlea 845 surrounding the cochlea opening 847. For example, the integrated tube sheath 864, or a portion thereof, could be configured to apply an outward compressive force on the portion of the cochlea surrounding the cochlea opening 847 to fluidically seal the cochlea opening 847 around the outer circumference of the tube sheath 864.

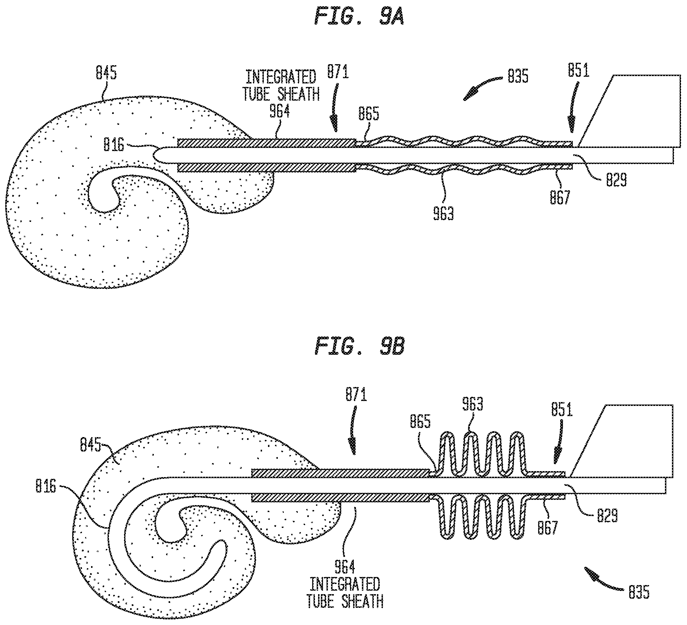

FIGs. 9A and 9B are cross-sectional views illustrating an alternative embodiment of an integrated tube sheath 964 that is similar to the embodiment of FIGs. 8A and 8B. However, in this embodiment, the flexible tube section 963 has a helical form (a helical surface profile) that could be produced, for example, by dip coating on a helical core and removed by a screwing/unscrewing motion.

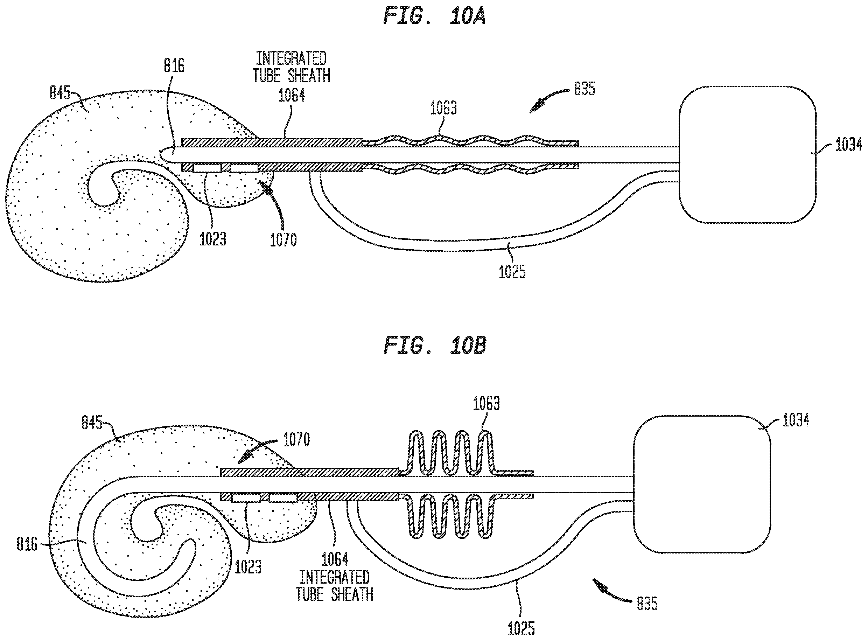

FIGs. 10A and 10B are cross-sectional views illustrating an alternative embodiment of an integrated tube sheath 1064 that is similar to the embodiment of FIGs. 9A and 9B, in terms of sealing the cochlea 845. That is, a helical flexible tube section (with a helical surface profile) 1063 is connected between a proximal end of the integrated tube sheath 1064 and a proximal end of the stimulation arrangement 835. However, in this embodiment, a distal portion 1070 of the tube sheath 1064 includes a plurality of electrodes 1023 disposed thereon. The electrodes 1023 are electrically connected to an implant body 1034 via a lead (e.g., one or more wires) 1025 and are configured to deliver stimulation signals (current) to the cochlea 845.

In other words, the integrated tube sheath 1064 is an electrically "active" sheath that is operable to stimulate the recipient. It is to be appreciated that these "active" embodiments of FIGs. 10A and 10B (i.e., one or more electrodes positioned on a distal portion of an integrated tube sheath that are electrically connected to the implant body) can also or alternatively be implemented with any of the other embodiments presented herein.

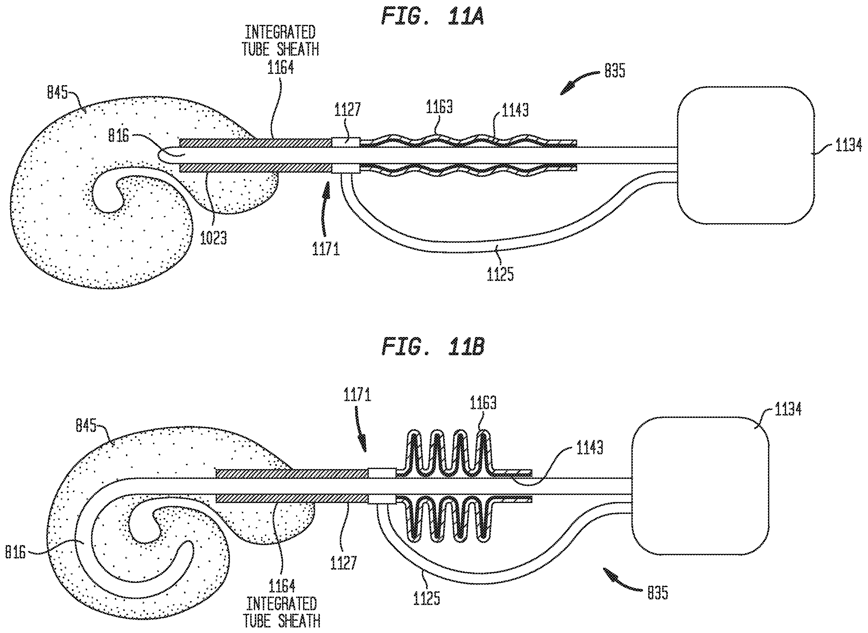

The above embodiments have primarily been described with reference to manual insertion of the stimulation assemblies. However, it is to be appreciated that embodiments presented herein can include an "actuated" sheath that is configured to automatically partially or fully insert a stimulation assembly into the cochlea (e.g., without manual intervention or with minimal manual intervention). For example, if an actuator such as a nitinol spring is incorporated into the tube sheath, then the associated stimulation assembly may be advanced without manual intervention. For example the nitinol shape memory transformation could be triggered by passing an electric current through it. This might be done in the surgery by connecting the nitinol to an external power source or the implant body. FIGs. 11A and 11B are cross-sectional views illustrating one example of an actuated sheath, in accordance with embodiments presented herein.

In the example of FIGs. 11A and 11B, the integrated tube sheath 1164 is similar to the embodiment of FIGs. 9A and 9B, in terms of sealing the cochlea 845. That is, a helical flexible tube section 1163 (tube with a helical surface profile) is connected between a proximal end of the integrated tube sheath 1064 and a proximal end of the stimulation arrangement 835. However, in this embodiment, a proximal portion 1171 of the tube sheath 1164 includes a plurality of electrodes 1127 disposed thereon. The electrodes 1127 are electrically connected to an implant body 1134 via a lead (e.g., one or more wires) 1125.

In the examples of FIGs. 11A and 11B, the helical flexible tube section 1163 comprises a nitinol member 1143 (e.g., nitinol coil spring). The nitinol member 1143 is configured to have current applied thereto via electrodes 1127. As the current is applied, the nitinol member 1143 contracts and advances the stimulation assembly 816 into the cochlea 845 as the spring contracted.

That is, as shown in FIG. 11A, the nitinol member 1143 has a first configuration/shape when the stimulation assembly 816 and tube sheath 1164 are first inserted into the cochlea 845. However, the implant body 1134 is configured to apply electrical signals to the nitinol member 1143 via the electrodes 1127. As shown in FIG. 11B, following application of the electrical signals, the nitinol member 1143 has a second configuration/shape that automatically advances/inserts the stimulation assembly 816 into the cochlea 845. Following insertion of the stimulation assembly 816, the nitinol member 1143 can retain the second configuration/shape to retain the position of the stimulation assembly 816 in the cochlea 845.

As noted elsewhere herein, the arrangements shown in FIGs. 2A-11B are merely illustrative and that techniques presented herein may be implemented with different arrangements. Also as noted elsewhere herein, embodiments presented herein have been primarily described with reference to an example auditory prosthesis system, namely a cochlear implant system. However, as noted above, it is to be appreciated that the techniques presented herein may be implemented by a variety of other types of implantable medical devices. For example, the techniques presented herein may be implemented by other auditory prostheses, such as acoustic hearing aids, middle ear auditory prostheses, bone conduction devices, direct acoustic stimulators, electro-acoustic prostheses, other electrically simulating auditory prostheses (e.g., auditory brain stimulators), *etc.* The techniques presented herein may also be implemented by tinnitus therapy devices, vestibular devices (e.g., vestibular implants), visual devices (i.e., bionic eyes), sensors, pacemakers, drug delivery systems, defibrillators, functional electrical stimulation devices, catheters, seizure devices (e.g., devices for monitoring and/or treating epileptic events), sleep apnea devices, electroporation devices, *etc.*

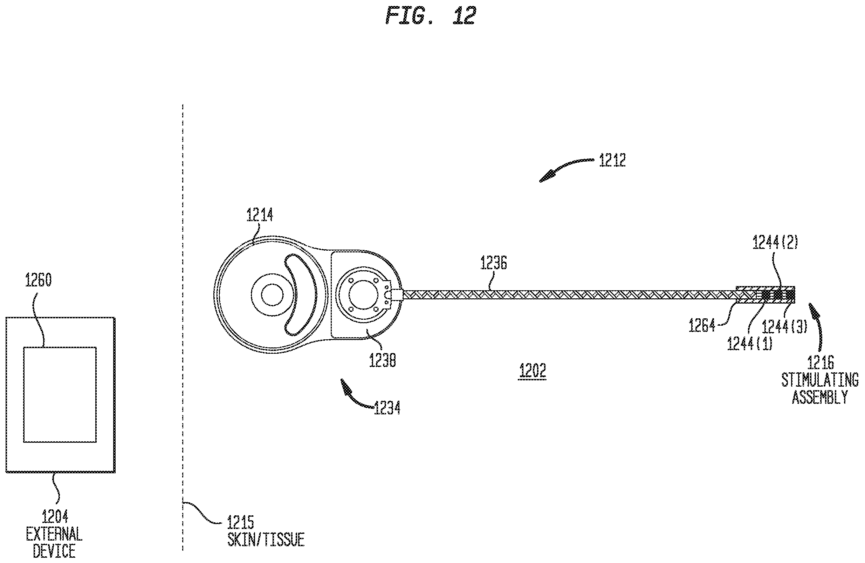

FIG. 12 illustrates an example vestibular stimulator system 1202, with which embodiments presented herein can be implemented. As shown, the vestibular stimulator system 1202 comprises an implantable component (vestibular stimulator) 1212 and an external device/component 1204 (e.g., external processing device, battery charger, remote control, *etc*.). The external device 1204 comprises a wireless power transmitter unit 1260 configured to transfer power (and potentially data) to the vestibular stimulator 1212,

The vestibular stimulator 1212 comprises an implant body (main module) 1234, a lead region 1236, and a stimulation assembly 1216, all configured to be implanted under the skin/tissue (tissue) 1215 of the recipient. The implant body 1234 generally comprises a hermetically-sealed housing 1238 in which RF interface circuitry, one or more rechargeable batteries, one or more processors, and a stimulator unit are disposed. The implant body 134 also includes an internal/implantable coil 1214 that is generally external to the housing 1238, but which is connected to the transceiver via a hermetic feedthrough (not shown).

The stimulation assembly 1216 comprises a plurality of electrodes 1244 disposed in a carrier member (e.g., a flexible silicone body). In this specific example, the stimulation assembly 1216 comprises three (3) stimulation electrodes, referred to as stimulation electrodes 1244(1), 1244(2), and 1244(3). The stimulation electrodes 1244(1), 1244(2), and 1244(3) function as an electrical interface for delivery of electrical stimulation signals to the recipient's vestibular system.

As shown, an integrated tube sheath 1264 is disposed around the stimulation assembly 1216 for use in insertion of the stimulation assembly 1216, for example, adjacent the recipient's otolith organs via, for example, the recipient's oval window. It is to be appreciated that this specific embodiment with three stimulation electrodes for stimulation assembly 1216 is merely illustrative and that the techniques presented herein may be used with stimulation assemblies having different numbers of stimulation electrodes, stimulation assemblies having different lengths, *etc.*

In accordance with the above, embodiments, the integrated tube sheath 1264 is configured to remain implanted in the recipient following of the stimulation assembly 1216 through the round window. The integrated tube sheath 1264 could remain within the round window following insertion and, accordingly, be configured in accordance with any of the above embodiments to fluidically seal the round window.

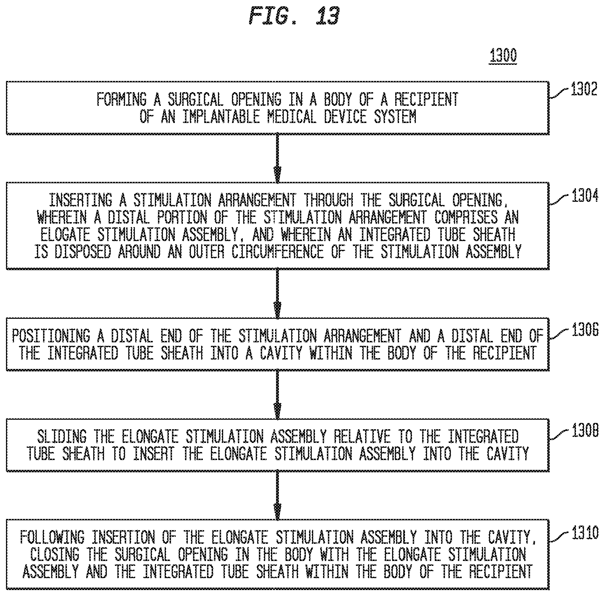

FIG. 13 is a flowchart of a method 1300, in accordance with embodiments presented herein. Method 1300 begins at 1302 where a surgical opening is formed in a body of a recipient of an implantable medical device system. At 1304, a stimulation arrangement is inserted through the surgical opening, wherein a distal portion of the stimulation arrangement comprises an elongate stimulation assembly, and wherein an integrated tube sheath is disposed around an outer circumference of the stimulation assembly. At 1306, a distal end of the stimulation arrangement and a distal end of the integrated tube sheath is positioned into a cavity within the body of the recipient. At 1308, the elongate stimulation assembly is slid relative to the integrated tube sheath to insert the elongate stimulation assembly into the cavity. At 1310, following insertion of the elongate stimulation assembly into the cavity, the surgical opening in the body is closed with the elongate stimulation assembly and the integrated tube sheath within the body of the recipient.

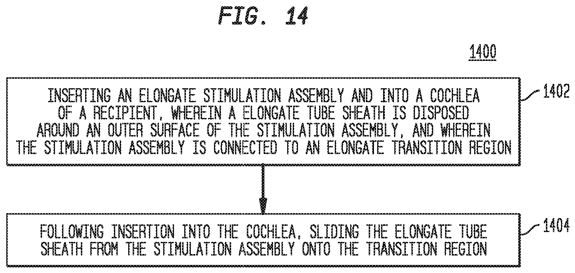

FIG. 14 is a flowchart of a method 1400, in accordance with embodiments presented herein. Method 1400 begins at 1402 where an elongate stimulation assembly is inserted into a cochlea of a recipient, wherein an elongate tube sheath is disposed around an outer surface of the elongate stimulation assembly, and wherein the elongate stimulation assembly is connected to an elongate transition region. At 1404, following insertion of the elongate stimulation assembly into the cochlea, the elongate tube sheath is slid from the elongate stimulation assembly onto the transition region.

As should be appreciated, while particular uses of the technology have been illustrated and discussed above, the disclosed technology can be used with a variety of devices in accordance with many examples of the technology. The above discussion is not meant to suggest that the disclosed technology is only suitable for implementation within systems akin to that illustrated in the figures. In general, additional configurations can be used to practice the processes and systems herein and/or some aspects described can be excluded without departing from the processes and systems disclosed herein.

This disclosure described some aspects of the present technology with reference to the accompanying drawings, in which only some of the possible aspects were shown. Other aspects can, however, be embodied in many different forms and should not be construed as limited to the aspects set forth herein. Rather, these aspects were provided so that this disclosure was thorough and complete and fully conveyed the scope of the possible aspects to those skilled in the art.

As should be appreciated, the various aspects (e.g., portions, components, etc.) described with respect to the figures herein are not intended to limit the systems and processes to the particular aspects described. Accordingly, additional configurations can be used to practice the methods and systems herein and/or some aspects described can be excluded without departing from the methods and systems disclosed herein.

Similarly, where steps of a process are disclosed, those steps are described for purposes of illustrating the present methods and systems and are not intended to limit the disclosure to a particular sequence of steps. For example, the steps can be performed in differing order, two or more steps can be performed concurrently, additional steps can be performed, and disclosed steps can be excluded without departing from the present disclosure. Further, the disclosed processes can be repeated.

Although specific aspects were described herein, the scope of the technology is not limited to those specific aspects. One skilled in the art will recognize other aspects or improvements that are within the scope of the present technology. Therefore, the specific structure, acts, or media are disclosed only as illustrative aspects. The scope of the technology is defined by the following claims and any equivalents therein.

It is also to be appreciated that the embodiments presented herein are not mutually exclusive and that the various embodiments may be combined with another in any of a number of different manners.

## Claims

1. An implantable medical device system (102), comprising:
a stimulation arrangement (135) comprising an elongate stimulation assembly (116) disposed at a distal end of the stimulation arrangement (135), wherein the elongate stimulation assembly (116) is configured to be inserted into a cochlea of a recipient; and
an integrated tube sheath (164) configured to be permanently disposed around and not removable from an outer circumference (149) of the stimulation arrangement (135).

2. The implantable medical device system of claim 1, wherein the integrated tube sheath (264) is configured to structurally support the elongate stimulation assembly (216) during insertion of the elongate stimulation assembly (216) into the cochlea, and wherein the integrated tube sheath (264) is slidably engaged with the outer circumference of the stimulation arrangement (235) for longitudinal advancement of the elongate stimulation assembly (216) relative to the integrated tube sheath (264) during insertion of the elongate stimulation assembly (216) into the cochlea,
or wherein at least a distal portion (270) of the integrated tube sheath (264) is configured to be positioned in the cochlea during insertion of the elongate stimulation assembly (216) into the cochlea, and wherein the distal portion (270) of the integrated tube sheath (264) is configured to be removed from the elongate stimulation assembly (216) following insertion of the elongate stimulation assembly (216) into the cochlea.

3. The implantable medical device system of claims 1 or 2, wherein the stimulation arrangement (235) comprises an elongate transition region (236) connected to a proximal end of the elongate stimulation assembly (216), and wherein, following insertion of the elongate stimulation assembly (216) into the cochlea, an inner surface (278) of the integrated tube sheath (264) is configured to be positioned abutting an outer surface (280) of the elongate transition region to eliminate spacing there between,
and optionally, wherein a portion of the elongate transition region (236) has an outer diameter (290) that is slightly larger than an inner diameter of the integrated tube sheath (264) such, that, when the inner surface (278) of the integrated tube sheath (264) is positioned abutting an outer surface (280) of the elongate transition region (236), the elongate transition region (236) is configured to apply an outward force on the integrated tube sheath (264),
or optionally, wherein at least one of the inner surface (278) of the integrated tube sheath (264) or the outer surface (280) of the elongate transition region (236) includes one or more antimicrobial substances (289).

4. The implantable medical device system of claims 1 or 2, further comprising:
an implant body (134) connected to a proximal end of the stimulation arrangement (135),
wherein, following insertion of the elongate stimulation assembly (116) into the cochlea, the integrated tube sheath (164) is configured to be positioned between the elongate stimulation assembly (116) and the implant body (134).

5. The implantable medical device system of claims 1 or 2, wherein following insertion of the elongate stimulation assembly (216) into the cochlea, a distal portion (270) of the integrated tube sheath (264) is configured to remain permanently disposed around a proximal region of the elongate stimulation assembly (216) within the cochlea.

6. The implantable medical device system of claim 5, wherein following insertion of the elongate stimulation assembly (216) into the cochlea, the distal portion (270) of the integrated tube sheath (264) is positioned over one or more electrodes (144) of the elongate stimulation assembly (116; 216), and wherein the distal portion (270) of the integrated tube sheath (264) has an electrically-transparent structure configured to allow stimulation current to flow from the one or more electrodes (144) to the recipient,
and optionally wherein the distal portion (270) of the integrated tube sheath (264) comprises at least one of a perforated or mesh-type construction,
or optionally, wherein the distal portion (270) of the integrated tube sheath (264) is at least partially bioresorbable so as to dissolve, in situ, within the cochlea,

7. The implantable medical device system of claim 5, wherein the integrated tube sheath (264) is configured to fluidically seal an opening in the cochlea through which the elongate stimulation assembly (216) and the integrated tube sheath (264) are inserted and extend through following insertion into the cochlea.

8. The implantable medical device system of claim 7, wherein the integrated tube sheath (364) comprises a slidable sealing member (394) configured to fluidically seal the opening around an outer surface (379) of the integrated tube sheath (364),
and optionally wherein the slidable sealing member (394) is configured to apply an inward compressive force on the outer surface (379) of the integrated tube sheath (364) to form a fluidic seal between the slidable sealing member (394) and the outer surface (379) of the integrated tube sheath (364), and wherein the slidable sealing member (394) is configured to be secured to the cochlea around the opening,
and optionally wherein the slidable sealing member (394) is configured to fill an interior of the integrated tube sheath (364) between an inner surface (378) of the integrated tube sheath (364) and the outer surface (379) of the elongate stimulation assembly (316) at the opening and to apply an inward compressive force on the outer surface (379) of the elongate stimulation assembly (316) to form a fluidic seal between the integrated tube sheath (364) and the elongate stimulation assembly (316) to fluidically seal the interior of the integrated tube sheath (364).

9. The implantable medical device system of claim 7, wherein the integrated tube sheath (464) comprises a fixed sealing member (494) comprising at least one annular member (495) extending about an circumference of an outer surface of the integrated tube sheath (464), wherein the at least one annular member (495) is configured to be secured to the cochlea adjacent to the opening to fluidically seal the opening through which the integrated tube sheath (464) extends,
and optionally wherein the integrated tube sheath (464) further comprises at least one inner annular member (496) extending about a circumference of an inner surface of the integrated tube sheath (464), wherein the at least one inner annular member (496) is configured for an interference fit with an outer surface of the elongate stimulation assembly (416) to fluidically seal an interior of the integrated tube sheath (464),
or optionally wherein the integrated tube sheath (464) further comprises one or more annular members (495) extending about a circumference of an outer surface of the elongate stimulation assembly (416), wherein the one or more annular members (495) are configured for an interference fit with an inner surface of the integrated tube sheath (464) to fluidically seal an interior of the integrated tube sheath (464).

10. The implantable medical device system of claim 7, wherein the integrated tube sheath (264) comprises a hydrogel outer layer configured to fluidically seal the opening around an outer surface of the integrated tube sheath (264),
or wherein the integrated tube sheath (264) comprises a hydrogel core configured to fluidically seal an interior of the integrated tube sheath (264) around the elongate stimulation assembly (216).

11. The implantable medical device system of claim 7, wherein the integrated tube sheath 664) comprises an annular stopper (657) configured to prevent over-insertion of the integrated tube sheath (664) into the cochlea, and wherein the annular stopper (657) is configured to fluidically seal the opening (647) around an outer surface (679) of the integrated tube sheath (664),
and optionally wherein the annular stopper (657) is resiliently flexible,
or optionally wherein the annular stopper (657) is configured to be secured to the cochlea to fluidically seal the opening (647) around an outer surface (679) of the integrated tube sheath (664).

12. The implantable medical device system of claim 7, further comprising a plug port (661) disposed as a proximal end of the integrated tube sheath (664), and a plug (659) configured to slide along the stimulation arrangement and configured to mechanically mate with the plug port (661) to fluidically seal the proximal end of the integrated tube sheath (664).

13. The implantable medical device system of claim 7, further comprising:
a proximally positioned flexible tube (863) having a distal end (865) connected to a proximal section of the integrated tube sheath (864) and a proximal end (867) connected to a proximal section (829) of the stimulation arrangement (835), wherein the flexible tube is configured to extend a certain distance relative to the elongate stimulation assembly (816) while maintaining a continuous fluidic barrier,
and optionally, wherein the flexible tube comprises a concertina-shaped tube with one or more waves,
or optionally wherein the flexible tube comprises a helical surface profile.

14. The implantable medical device system of claim 5, further comprising:
a stimulator unit (142); and
one or more electrodes (144) positioned on the distal portion of the integrated tube sheath (164) electrically connected to the stimulator unit (142),
or wherein the integrated tube sheath (164) is an actuated sheath configured to automatically partially or fully insert the elongate stimulation assembly (116) into the cochlea.

15. The implantable medical device system of claims 1 or 2, wherein the stimulation arrangement (235) includes an elongate embedded stiffener disposed proximal to the elongate stimulation assembly (216),
or wherein the integrated tube sheath (264) comprises a handle (282) disposed at a proximal end of the integrated tube sheath (264),
or wherein the integrated tube sheath (264) comprises a fixation feature for securing the integrated tube sheath (264) to the recipient.

## Patentansprüche

1. . Implantierbares medizinisches Vorrichtungssystem (102), umfassend:
eine Stimulationsanordnung (135), die eine längliche Stimulationsanordnung (116) umfasst, die an einem distalen Ende der Stimulationsanordnung (135) angeordnet ist, wobei die längliche Stimulationsanordnung (116) so ausgebildet ist, dass sie in eine Cochlea eines Empfängers eingeführt werden kann; und
eine integrierte Schlauchhülle (164), die so ausgebildet ist, dass sie dauerhaft um einen Außenumfang (149) der Stimulationsanordnung (135) herum angeordnet und von diesem nicht entfernbar ist.

2. . Implantierbares medizinisches Vorrichtungssystem nach Anspruch 1, wobei die integrierte Schlauchhülle (264) so ausgebildet ist, dass sie die längliche Stimulationsanordnung (216) während des Einführens der länglichen Stimulationsanordnung (216) in die Cochlea strukturell stützt, und wobei die integrierte Schlauchhülle (264) bei dem Einführen der länglichen Stimulationsanordnung (216) in die Cochlea verschiebbar mit dem Außenumfang der Stimulationsanordnung (235) zu dem Längsvorschub der länglichen Stimulationsanordnung (216) relativ zu der integrierten Schlauchhülle (264) in Eingriff steht,
oder wobei mindestens ein distaler Abschnitt (270) der integrierten Schlauchhülle (264) so ausgebildet ist, dass er während des Einführens der länglichen Stimulationsanordnung (216) in die Cochlea in der Cochlea positioniert werden kann, und wobei der distale Abschnitt (270) der integrierten Schlauchhülle (264) so ausgebildet ist, dass er nach dem Einführen der länglichen Stimulationsanordnung (216) in die Cochlea aus der länglichen Stimulationsanordnung (216) entfernt werden kann.

3. . Implantierbares medizinisches Vorrichtungssystem nach den Ansprüchen 1 oder 2, wobei die Stimulationsanordnung (235) einen länglichen Übergangsbereich (236) umfasst, der mit einem proximalen Ende der länglichen Stimulationsanordnung (216) verbunden ist, und wobei nach dem Einführen der länglichen Stimulationsanordnung (216) in die Cochlea eine Innenfläche (278) der integrierten Schlauchhülle (264) so ausgebildet ist, dass sie anliegend an einer Außenfläche (280) des länglichen Übergangsbereichs positioniert werden kann, um einen Abstand dazwischen zu vermeiden,
und wobei wahlweise ein Abschnitt des länglichen Übergangsbereichs (236) einen Außendurchmesser (290) aufweist, der etwas größer ist als ein Innendurchmesser der integrierten Schlauchhülle (264), so dass, wenn die Innenfläche (278) der integrierten Schlauchhülle (264) anliegend an einer Außenfläche (280) des länglichen Übergangsbereichs (236) positioniert ist, der längliche Übergangsbereich (236) so ausgebildet ist, dass er eine nach außen gerichtete Kraft auf die integrierte Schlauchhülle (264) ausübt,
oder wobei wahlweise mindestens eine der Innenfläche (278) der integrierten Schlauchhülle (264) oder der Außenfläche (280) des länglichen Übergangsbereichs (236) eine oder mehrere antimikrobielle Substanzen (289) einschließt.

4. . Implantierbares medizinisches Vorrichtungssystem nach den Ansprüchen 1 oder 2, weiter umfassend:
einen Implantatkörper (134), der mit einem proximalen Ende der Stimulationsanordnung (135) verbunden ist, wobei nach dem Einführen der länglichen Stimulationsanordnung (116) in die Cochlea die integrierte Schlauchhülle (164) so ausgebildet ist, dass sie zwischen der länglichen Stimulationsanordnung (116) und dem Implantatkörper (134) positioniert werden kann.

5. . Implantierbares medizinisches Vorrichtungssystem nach den Ansprüchen 1 oder 2, wobei nach dem Einführen der länglichen Stimulationsanordnung (216) in die Cochlea ein distaler Abschnitt (270) der integrierten Schlauchhülle (264) so ausgebildet ist, dass er dauerhaft um einen proximalen Bereich der länglichen Stimulationsanordnung (216) innerhalb der Cochlea angeordnet bleibt.

6. . Implantierbares medizinisches Vorrichtungssystem nach Anspruch 5, wobei nach dem Einführen der länglichen Stimulationsanordnung (216) in die Cochlea der distale Abschnitt (270) der integrierten Schlauchhülle (264) über einer oder mehreren Elektroden (144) der länglichen Stimulationsanordnung (116; 216) positioniert ist, und wobei der distale Abschnitt (270) der integrierten Schlauchhülle (264) eine elektrisch transparente Struktur aufweist, die so ausgebildet ist, dass ein Stimulationsstrom von der einen oder den mehreren Elektroden (144) zu dem Empfänger fließen kann,
und wobei wahlweise der distale Abschnitt (270) der integrierten Schlauchhülle (264) mindestens einen von einer perforierten oder netzartigen Konstruktion umfasst,
oder wobei wahlweise der distale Abschnitt (270) der integrierten Schlauchhülle (264) mindestens teilweise bioresorbierbar ist, um sich in situ innerhalb der Cochlea aufzulösen,

7. . Implantierbares medizinisches Vorrichtungssystem nach Anspruch 5, wobei die integrierte Schlauchhülle (264) so ausgebildet ist, dass sie eine Öffnung in der Cochlea fluidisch abdichtet, durch die die längliche Stimulationsanordnung (216) und die integrierte Schlauchhülle (264) eingeführt werden und sich nach dem Einführen in die Cochlea durch diese erstrecken.

8. . Implantierbares medizinisches Vorrichtungssystem nach Anspruch 7, wobei die integrierte Schlauchhülle (364) ein verschiebbares Dichtungselement (394) umfasst, das so ausgebildet ist, dass es die Öffnung um eine Außenfläche (379) der integrierten Schlauchhülle (364) fluidisch abdichtet,
und wobei wahlweise das verschiebbare Dichtungselement (394) so ausgebildet ist, dass es eine nach innen gerichtete Druckkraft auf die Außenfläche (379) der integrierten Schlauchhülle (364) ausübt, um eine fluidische Abdichtung zwischen dem verschiebbaren Dichtungselement (394) und der Außenfläche (379) der integrierten Schlauchhülle (364) zu bilden, und wobei das verschiebbare Dichtungselement (394) so ausgebildet ist, dass es um die Öffnung herum an der Cochlea befestigt werden kann,
und wobei wahlweise das verschiebbare Dichtungselement (394) so ausgebildet ist, dass es einen Innenraum der integrierten Schlauchhülle (364) zwischen einer Innenfläche (378) der integrierten Schlauchhülle (364) und der Außenfläche (379) der länglichen Stimulationsanordnung (316) an der Öffnung füllt und dass es eine nach innen gerichtete Druckkraft auf die Außenfläche (379) der länglichen Stimulationsanordnung (316) ausübt, um eine fluidische Abdichtung zwischen der integrierten Schlauchhülle (364) und der länglichen Stimulationsanordnung (316) zu bilden, um das Innere der integrierten Schlauchhülle (364) fluidisch abzudichten.

9. . Implantierbares medizinisches Vorrichtungssystem nach Anspruch 7, wobei die integrierte Schlauchhülle (464) ein festes Dichtungselement (494) umfasst, das mindestens ein ringförmiges Element (495) umfasst, das sich um einen Umfang einer Außenfläche der integrierten Schlauchhülle (464) erstreckt, wobei das mindestens eine ringförmige Element (495) so ausgebildet ist, dass es an der Cochlea neben der Öffnung befestigt werden kann, um die Öffnung, durch die sich die integrierte Schlauchhülle (464) erstreckt, fluidisch abzudichten,
und wobei wahlweise die integrierte Schlauchhülle (464) weiter mindestens ein inneres ringförmiges Element (496) umfasst, das sich um einen Umfang einer Innenfläche der integrierten Schlauchhülle (464) erstreckt, wobei das mindestens eine innere ringförmige Element (496) für eine Presspassung mit einer Außenfläche der länglichen Stimulationsanordnung (416) ausgebildet ist, um ein Inneres der integrierten Schlauchhülle (464) fluidisch abzudichten,
oder wobei wahlweise die integrierte Schlauchhülle (464) weiter ein oder mehrere ringförmige Elemente (495) umfasst, die sich um einen Umfang einer Außenfläche der länglichen Stimulationsanordnung (416) erstrecken, wobei das eine oder die mehreren ringförmigen Elemente (495) für eine Presspassung mit einer Innenfläche der integrierten Schlauchhülle (464) ausgebildet sind, um ein Inneres der integrierten Schlauchhülle (464) fluidisch abzudichten.

10. . Implantierbares medizinisches Vorrichtungssystem nach Anspruch 7, wobei die integrierte Schlauchhülle (264) eine Hydrogel-Außenschicht umfasst, die so ausgebildet ist, dass sie die Öffnung um eine Außenfläche der integrierten Schlauchhülle (264) fluidisch abdichtet,
oder wobei die integrierte Schlauchhülle (264) einen Hydrogelkern umfasst, der so ausgebildet ist, dass er ein Inneres der integrierten Schlauchhülle (264) um die längliche Stimulationsanordnung (216) herum fluidisch abdichtet.

11. . Implantierbares medizinisches Vorrichtungssystem nach Anspruch 7, wobei die integrierte Schlauchhülle (664) einen ringförmigen Anschlag (657) umfasst, der so ausgebildet ist, dass er ein zu tiefes Einführen der integrierten Schlauchhülle (664) in die Cochlea verhindert, und wobei der ringförmige Anschlag (657) so ausgebildet ist, dass er die Öffnung (647) um eine Außenfläche (679) der integrierten Schlauchhülle (664) herum fluidisch abdichtet,
und wobei wahlweise der ringförmige Anschlag (657) elastisch flexibel ist,
oder wobei wahlweise der ringförmige Anschlag (657) so ausgebildet ist, dass er an der Cochlea befestigt werden kann, um die Öffnung (647) um eine Außenfläche (679) der integrierten Schlauchhülle (664) herum fluidisch abzudichten.

12. . Implantierbares medizinisches Vorrichtungssystem nach Anspruch 7, weiter umfassend einen Stopfenanschluss (661), der als proximales Ende der integrierten Schlauchhülle (664) angeordnet ist, und einen Stopfen (659), der so ausgebildet ist, dass er entlang der Stimulationsanordnung gleitet, und so ausgebildet ist, dass er mechanisch mit dem Stopfenanschluss (661) zusammenpasst, um das proximale Ende der integrierten Schlauchhülle (664) fluidisch abzudichten.

13. . Implantierbares medizinisches Vorrichtungssystem nach Anspruch 7, weiter umfassend:
einen proximal positionierten flexiblen Schlauch (863), der ein distales Ende (865) aufweist, das mit einem proximalen Abschnitt der integrierten Schlauchhülle (864) verbunden ist, und ein proximales Ende (867), das mit einem proximalen Abschnitt (829) der Stimulationsanordnung (835) verbunden ist, wobei der flexible Schlauch so ausgebildet ist, dass er sich über einen bestimmten Abstand relativ zu der länglichen Stimulationsanordnung (816) erstreckt, während er eine kontinuierliche Fluidbarriere aufrechterhält,
und wobei wahlweise der flexible Schlauch einen ziehharmonikaförmigen Schlauch mit einer oder mehreren Wellen umfasst,
oder wobei wahlweise der flexible Schlauch ein spiralförmiges Oberflächenprofil umfasst.

14. . Implantierbares medizinisches Vorrichtungssystem nach Anspruch 5, weiter umfassend:
eine Stimulatoreinheit (142); und
eine oder mehrere Elektroden (144), die auf dem distalen Abschnitt der integrierten Schlauchhülle (164) positioniert sind, die elektrisch mit der Stimulatoreinheit (142) verbunden sind,
oder wobei die integrierte Schlauchhülle (164) eine betätigte Hülle ist, die so ausgebildet ist, dass sie die längliche Stimulationsanordnung (116) automatisch teilweise oder vollständig in die Cochlea einführt.

15. . Implantierbares medizinisches Vorrichtungssystem nach den Ansprüchen 1 oder 2, wobei die Stimulationsanordnung (235) eine längliche, eingebettete Versteifung einschließt, die proximal zu der länglichen Stimulationsanordnung (216) angeordnet ist,
oder wobei die integrierte Schlauchhülle (264) einen Griff (282) umfasst, der an einem proximalen Ende der integrierten Schlauchhülle (264) angeordnet ist,
oder wobei die integrierte Schlauchhülle (264) eine Fixierungsvorrichtung zum Befestigen der integrierten Schlauchhülle (264) an dem Empfänger umfasst.

## Revendications

1. Système (102) de dispositif médical implantable, comprenant :
un agencement de stimulation (135) comprenant un ensemble de stimulation allongé (116) disposé à une extrémité distale de l'agencement de stimulation (135), dans lequel l'ensemble de stimulation allongé (116) est configuré pour être inséré dans une cochlée d'un receveur ; et
une gaine tubulaire intégrée (164) configurée pour être disposée de manière permanente autour et pour ne pas pouvoir être retirée d'une circonférence externe (149) de l'agencement de stimulation (135).

2. Système de dispositif médical implantable selon la revendication 1, dans lequel la gaine tubulaire intégrée (264) est configurée pour soutenir structuralement l'ensemble de stimulation allongé (216) pendant l'insertion de l'ensemble de stimulation allongé (216) dans la cochlée, et dans lequel la gaine tubulaire intégrée (264) est en prise coulissante avec la circonférence externe de l'agencement de stimulation (235) pour l'avancement longitudinal de l'ensemble de stimulation allongé (216) par rapport à la gaine tubulaire intégrée (264) pendant l'insertion de l'ensemble de stimulation allongé (216) dans la cochlée, ou dans lequel au moins une partie distale (270) de la gaine tubulaire intégrée (264) est configurée pour être positionnée dans la cochlée lors de l'insertion de l'ensemble de stimulation allongé (216) dans la cochlée, et dans lequel la partie distale (270) de la gaine tubulaire intégrée (264) est configurée pour être retirée de l'ensemble de stimulation allongé (216) après insertion de l'ensemble de stimulation allongé (216) dans la cochlée.

3. Système de dispositif médical implantable selon les revendications 1 ou 2, dans lequel l'agencement de stimulation (235) comprend une région de transition allongée (236) reliée à une extrémité proximale de l'ensemble de stimulation allongé (216), et dans lequel, après insertion de l'ensemble de stimulation allongé (216) dans la cochlée, une surface interne (278) de la gaine tubulaire intégrée (264) est configurée pour être positionnée en butée contre une surface externe (280) de la région de transition allongée afin d'éliminer l'espacement entre les deux, et facultativement dans lequel une partie de la région de transition allongée (236) présente un diamètre externe (290) qui est légèrement plus grand qu'un diamètre interne de la gaine tubulaire intégrée (264), de sorte que, lorsque la surface interne (278) de la gaine tubulaire intégrée (264) est positionnée en butée contre une surface externe (280) de la région de transition allongée (236), la région de transition allongée (236) est configurée pour appliquer une force vers l'extérieur sur la gaine tubulaire intégrée (264), ou facultativement dans lequel au moins l'une de la surface interne (278) de la gaine tubulaire intégrée (264) ou la surface externe (280) de la région de transition allongée (236) inclut une ou plusieurs substances antimicrobiennes (289).

4. Système de dispositif médical implantable selon les revendications 1 ou 2, comprenant en outre :
un corps d'implant (134) relié à une extrémité proximale de l'agencement de stimulation (135), dans lequel, après insertion de l'ensemble de stimulation allongé (116) dans la cochlée, la gaine tubulaire intégrée (164) est configurée pour être positionnée entre l'ensemble de stimulation allongé (116) et le corps d'implant (134).

5. Système de dispositif médical implantable selon les revendications 1 ou 2, dans lequel, après insertion de l'ensemble de stimulation allongé (216) dans la cochlée, une partie distale (270) de la gaine tubulaire intégrée (264) est configurée pour rester disposée de manière permanente autour d'une région proximale de l'ensemble de stimulation allongé (216) à l'intérieur de la cochlée.

6. Système de dispositif médical implantable selon la revendication 5, dans lequel après insertion de l'ensemble de stimulation allongé (216) dans la cochlée, la partie distale (270) de la gaine tubulaire intégrée (264) est positionnée sur une ou plusieurs électrodes (144) de l'ensemble de stimulation allongé (116 ; 216), et dans lequel la partie distale (270) de la gaine tubulaire intégrée (264) présente une structure électriquement transparente configurée pour permettre à un courant de stimulation de circuler depuis les une ou plusieurs électrodes (144) vers le receveur, et facultativement dans lequel la partie distale (270) de la gaine tubulaire intégrée (264) comprend au moins l'une d'une construction perforée ou de type maillage, ou facultativement dans lequel la partie distale (270) de la gaine tubulaire intégrée (264) est au moins partiellement biorésorbable de manière à se dissoudre, in situ, à l'intérieur de la cochlée.

7. Système de dispositif médical implantable selon la revendication 5, dans lequel la gaine tubulaire intégrée (264) est configurée pour sceller fluidiquement une ouverture dans la cochlée à travers laquelle l'ensemble de stimulation allongé (216) et la gaine tubulaire intégrée (264) sont insérés et s'étendent après insertion dans la cochlée.

8. Système de dispositif médical implantable selon la revendication 7, dans lequel la gaine tubulaire intégrée (364) comprend un organe d'étanchéité coulissant (394) configuré pour sceller fluidiquement l'ouverture autour d'une surface externe (379) de la gaine tubulaire intégrée (364),
et facultativement dans lequel l'organe d'étanchéité coulissant (394) est configuré pour appliquer une force de compression vers l'intérieur sur la surface externe (379) de la gaine tubulaire intégrée (364) pour former un joint fluidique entre l'organe d'étanchéité coulissant (394) et la surface externe (379) de la gaine tubulaire intégrée (364), et dans lequel l'organe d'étanchéité coulissant (394) est configuré pour être attaché à la cochlée autour de l'ouverture,
et facultativement dans lequel l'organe d'étanchéité coulissant (394) est configuré pour remplir un intérieur de la gaine tubulaire intégrée (364) entre une surface interne (378) de la gaine tubulaire intégrée (364) et la surface externe (379) de l'ensemble de stimulation allongé (316) au niveau de l'ouverture et pour appliquer une force de compression vers l'intérieur sur la surface externe (379) de l'ensemble de stimulation allongé (316) pour former un joint fluidique entre la gaine tubulaire intégrée (364) et l'ensemble de stimulation allongé (316) pour sceller fluidiquement l'intérieur de la gaine tubulaire intégrée (364).

9. Système de dispositif médical implantable selon la revendication 7, dans lequel la gaine tubulaire intégrée (464) comprend un organe d'étanchéité fixe (494) comprenant au moins un organe annulaire (495) s'étendant autour d'une circonférence d'une surface externe de la gaine tubulaire intégrée (464), dans lequel l'au moins un organe annulaire (495) est configuré pour être attaché à la cochlée de manière adjacente à l'ouverture pour sceller fluidiquement l'ouverture à travers laquelle s'étend la gaine tubulaire intégrée (464),
et facultativement dans lequel la gaine tubulaire intégrée (464) comprend en outre au moins un organe annulaire interne (496) s'étendant autour d'une circonférence d'une surface interne de la gaine tubulaire intégrée (464), dans lequel l'au moins un organe annulaire interne (496) est configuré pour un ajustement avec serrage avec une surface externe de l'ensemble de stimulation allongé (416) pour sceller fluidiquement un intérieur de la gaine tubulaire intégrée (464),
ou facultativement dans lequel la gaine tubulaire intégrée (464) comprend en outre un ou plusieurs organes annulaires (495) s'étendant autour d'une circonférence d'une surface externe de l'ensemble de stimulation allongé (416), dans lequel les un ou plusieurs organes annulaires (495) sont configurés pour un ajustement avec serrage avec une surface interne de la gaine tubulaire intégrée (464) pour sceller fluidiquement un intérieur de la gaine tubulaire intégrée (464).

10. Système de dispositif médical implantable selon la revendication 7, dans lequel la gaine tubulaire intégrée (264) comprend une couche externe d'hydrogel configurée pour sceller fluidiquement l'ouverture autour d'une surface externe de la gaine tubulaire intégrée (264),
ou dans lequel la gaine tubulaire intégrée (264) comprend un noyau d'hydrogel configuré pour sceller fluidiquement un intérieur de la gaine tubulaire intégrée (264) autour de l'ensemble de stimulation allongé (216).

11. Système de dispositif médical implantable selon la revendication 7, dans lequel la gaine tubulaire intégrée (664) comprend une butée annulaire (657) configurée pour empêcher une insertion excessive de la gaine tubulaire intégrée (664) dans la cochlée, et dans lequel la butée annulaire (657) est configurée pour sceller fluidiquement l'ouverture (647) autour d'une surface externe (679) de la gaine tubulaire intégrée (664),
et facultativement dans lequel la butée annulaire (657) est élastiquement flexible, ou facultativement dans lequel la butée annulaire (657) est configurée pour être attachée à la cochlée pour sceller fluidiquement l'ouverture (647) autour d'une surface externe (679) de la gaine tubulaire intégrée (664).

12. Système de dispositif médical implantable selon la revendication 7, comprenant en outre un orifice (661) de bouchon disposé comme extrémité proximale de la gaine tubulaire intégrée (664), et un bouchon (659) configuré pour coulisser le long de l'agencement de stimulation et configuré pour se coupler mécaniquement avec l'orifice (661) de bouchon pour sceller fluidiquement l'extrémité proximale de la gaine tubulaire intégrée (664).

13. Système de dispositif médical implantable selon la revendication 7, comprenant en outre :
un tube flexible (863) positionné proximalement présentant une extrémité distale (865) reliée à une section proximale de la gaine tubulaire intégrée (864) et une extrémité proximale (867) reliée à une section proximale (829) de l'agencement de stimulation (835), dans lequel le tube flexible est configuré pour s'étendre sur une certaine distance par rapport à l'ensemble de stimulation allongé (816) tout en maintenant une barrière fluidique continue,
et facultativement dans lequel le tube flexible comprend un tube en forme d'accordéon avec une ou plusieurs ondulations,
ou facultativement dans lequel le tube flexible comprend un profil de surface hélicoïdal.

14. Système de dispositif médical implantable selon la revendication 5, comprenant en outre :
une unité de stimulateur (142) ; et
une ou plusieurs électrodes (144) positionnées sur la partie distale de la gaine tubulaire intégrée (164) en connexion électrique avec l'unité de stimulateur (142),
ou dans lequel la gaine tubulaire intégrée (164) est une gaine actionnée configurée pour insérer automatiquement partiellement ou complètement l'ensemble de stimulation allongé (116) dans la cochlée.

15. Système de dispositif médical implantable selon les revendications 1 ou 2, dans lequel l'agencement de stimulation (235) inclut un raidisseur incorporé allongé disposé de manière proximale à l'ensemble de stimulation allongé (216),
ou dans lequel la gaine tubulaire intégrée (264) comprend une poignée (282) disposée à une extrémité proximale de la gaine tubulaire intégrée (264),
ou dans lequel la gaine tubulaire intégrée (264) comprend un élément de fixation pour attacher la gaine tubulaire intégrée (264) au receveur.
